# EUROPÄISCHE PATENTSCHRIFT

(11) **EP 2 898 055 B1**
(45) Veröffentlichungstag und Bekanntmachung des Hinweises auf die Patenterteilung: **23.05.2018**
(21) Anmeldenummer: 14705706.1
(22) Anmeldetag: 04.02.2014
(51) Int. Cl.: C12M 1/00, C12M 3/00, C12M 1/34, C12M 1/06, F16M 11/08, F16M 11/04, B65G 67/04

(54) **BEHÄLTER, TRANSPORTVORRICHTUNG, VERWENDUNG UND VERFAHREN**
CONTAINER, TRANSPORT DEVICE, USE AND METHOD
RÉCIPIENT, DISPOSITIF DE TRANSPORT, UTILISATION ET PROCÉDÉ

(30) Priorität: 05.02.2013 DE 102013002091
(43) Veröffentlichungstag der Anmeldung: 29.07.2015
(73) Patentinhaber: Sartorius Stedim Biotech GmbH, 37079 Göttingen (DE)
(72) Erfinder: HUSEMANN, Bernward, 37083 Göttingen (DE); KAHLERT, Wolfgang, 34327 Körle (DE); CHAUSSIN, Sebastien, 13400 Aubagne (FR); SEITZ, Reiner, 3506 Helsa (DE)
(74) Vertreter: Müller-Boré & Partner Patentanwälte PartG mbB
(86) Internationale Anmeldenummer: PCT/EP2014/000284
(87) Internationale Veröffentlichungsnummer: WO 2014/121918

(56) Entgegenhaltungen:
- US-A1- 2007 224 676
- US-A1- 2010 255 582
- US-A1- 2011 038 222
- US-A1- 2012 282 688

## Beschreibung

Die Erfindung betrifft einen Behälter, eine Transportvorrichtung zum Heben und Transportieren des Behälters und ein Verfahren zum Einsetzen des Behälters in eine Haltevorrichtung.

Insbesondere in der pharmazeutischen und biotechnologischen Industrie werden bereichsweise flexible Behälter, wie zum Beispiel Beutel, als Bioreaktorbehälter für die Prozeßführung bzw. Lagerung verwendet. Die Behälter können dabei ein Volumen von 1000 Litern oder mehr fassen und sind daher zum Transport platzsparend zusammengefaltet. Zum Gebrauch werden die Behälter in einer im wesentlichen starren Haltevorrichtung angeordnet, welche die Wandung des Behälters stützt, so daß der nach dem Befüllen des Behälters wirkende Druck auf die Wandung des Behälter durch die Haltevorrichtung aufgenommen wird. Die Wandung des Behälters als solche ist in der Regel nicht stark genug ausgebildet, um den Inhalt des Behälters nach dem Befüllen zu halten. Mit anderen Worten würde der Behälter während oder nach dem Befüllen außerhalb der Haltevorrichtung platzen. Aufgrund ihrer Größe und ihres Gewichtes sind die Behälter nur schwer oder gar nicht mehr manuell handhabbar.

Des Weiteren offenbart die US 2010 /025 5582 A1 eine Vorrichtung und ein Verfahren zum Aussähen und Züchten von Zellen auf einer Probe. Die Vorrichtung umfasst eine Kammer, in der das Volumen der Kammer eingestellt werden kann, ohne dass die Dichtung oder die Sterilität der Kammer beeinträchtigt wird. Die Vorrichtung ermöglicht das Aussäen der Zellen in einem reduzierten Volumen und das Kultivieren von Zellen in einem vergrößerten Volumen. Ferner ermöglicht die Vorrichtung während des Aussähens, Kultivierens oder Transport der Probe, die Anwendung von Kräften, Dehnungen und Drehmomenten auf die Probe.

Es ist daher eine Aufgabe der Erfindung, einen Behälter, eine Transportvorrichtung zum Heben und Transportieren des Behälters und ein Verfahren zum Einsetzen des Behälters in eine Haltevorrichtung bereitzustellen, wobei eine verbesserte Handhabung des Behälters ermöglicht wird.

Die Aufgabe wird durch einen Behälter mit den Merkmalen des Anspruchs 1, eine Verwendung mit den Merkmalen des Anspruchs 10, eine Transportvorrichtung mit den Merkmalen des Anspruchs 11 und ein Verfahren mit den Merkmalen des Anspruchs 12 gelöst. Bevorzugte Ausführungsformen sind Gegenstand der abhängigen Ansprüche.

### Behälter gemäß einem Aspekt

Ein Aspekt betrifft einen Behälter zum Einsetzen in eine Haltevorrichtung umfassend:
- eine zumindest bereichsweise flexible Wandung, welche in einem Transportzustand entgegen einer Streckrichtung zumindest bereichsweise verkürzt ist;
- eine mit der Wandung verbundene Handhabungseinrichtung, welche ausgelegt ist, mit einer komplementären Handhabungseinrichtung einer Transportvorrichtung in Eingriff zu gelangen;
- eine Sicherungseinrichtung, welche im Transportzustand ein Strecken der Wandung des Behälters entlang der Streckrichtung hemmt, wobei der Behälter zumindest bereichsweise entlang der Streckrichtung verlängerbar ist, wenn die Sicherungseinrichtung gelöst ist.

Vorteilhafterweise erlaubt die Sicherungseinrichtung einen Transport bzw. ein Halten und Heben des Behälters, wobei der Behälter im Transportzustand, also im verkürzten Zustand, verbleibt und daher aufgrund der geringeren Größe verglichen einem Anordnungszustand, in welchem der Behälter gestreckt ist, einfacher zu handhaben ist. Weiter vorteilhafterweise kann die Wandung des Behälters durch die Sicherungseinrichtung mechanisch stabilisiert werden, insbesondere wenn der Behälter in einer bevorzugten Ausführungsform gefüllt ist, beispielsweise mit einem Fluid und/oder einem pulverförmigen, körnigen bzw. rieselfähigen Feststoff.

Der Begriff "Behälter" im Sinne der Anmeldung umfaßt Behälter, insbesondere Bioreaktorbehälter, mit bereichsweise flexiblen Wänden, die beispielsweise zur Aufnahme, zum Mischen, zur Speicherung und zum Spenden von Medien, insbesondere sterilen Medien, dienen können.

Der Behälter umfaßt dazu eine fluiddichte Wandung, welche zumindest bereichsweise flexibel ist. Insbesondere weist der Behälter eine Wandung auf, welche aus einer Folie bzw. aus einem Verbund bzw. Laminat mehrerer Folien ausgebildet ist. Mit anderen Worten kann der Behälter als Beutel ausgebildet sein. Der Behälter ist aufgrund seiner variablen Erstreckung entlang der Streckrichtung bzw. seiner Verkürzbarkeit und Streckbarkeit volumenvariabel. Insbesondere kann der Behälter im gestreckten Anordnungszustand ein Volumen aufweisen, welches um einem Faktor größer als etwa 5, bevorzugt größer als etwa 10 und insbesondere größer als etwa 20 ist, als das Volumen des Behälters im verkürzten Transportzustand.

Mit anderen Worten kann das Innenvolumen des Behälters im Transportzustand minimal sein und sich mit dem Befüllen des Behälters vergrößern und mit dem Entleeren wieder verringern. Zum Befüllen und Entleeren kann der Behälter zumindest eine Behälteröffnung aufweisen. Die zumindest eine Behälteröffnung ist bevorzugt als Anschluß bzw. Verbinder ausgebildet, so daß Fluidleitungen mittels des zumindest einen Anschlusses in einfacher Weise mit dem Behälter verbindbar sind.

Bevorzugt ist der Behälter im wesentlichen entlang genau einer Streckrichtung streckbar bzw. verkürzbar. Mit anderen Worten bleibt die Erstreckung des Behälters in zwei senkrecht zueinander stehenden Richtungen, welche im wesentlichen senkrecht zur Streckrichtung orientiert sind, beim Übergang vom Transportzustand zum Anordnungszustand im wesentlichen konstant. Insbesondere kann die Länge der Erstreckung in diesen beiden Richtungen im Anordnungszustand etwa 50 Prozent bis etwa 150 Prozent der Länge der Erstreckung im Transportzustand betragen. Dagegen kann die Länge entlang der Streckrichtung im Anordnungszustand etwa größer als etwa 200 Prozent, bevorzugt größer als etwa 500 Prozent, insbesondere größer als etwa 1000 Prozent, der Länge in der Streckrichtung im Transportzustand betragen.

Der Behälter weist eine Handhabungseinrichtung auf, welche mechanisch starrer ausgebildet ist als die flexible Wandung des Behälters. Vorteilhafterweise kann die komplementäre Handhabungseinrichtung der Transportvorrichtung den Behälter im Bereich der Handhabungseinrichtung greifen ohne die flexible Wandung des Behälters zu verformen. Weiter vorteilhafterweise wird dadurch die flexible Wandung des Behälters nicht mechanisch beansprucht, so daß die Gefahr verringert wird, durch den Transport und die Handhabung des Behälters die Wandung zu beschädigen.

Der Eingriff zwischen der komplementären Handhabungseinrichtung und der Handhabungseinrichtung des Behälters kann einen Formschluß bzw. einen Reibschluß bzw. einen Kraftschluß beinhalten, so daß ein Lösen des Behälters von der Transportvorrichtung gehemmt bzw. verhindert ist. Bevorzugt kann die komplementäre Handhabungseinrichtung die Handhabungseinrichtung des Behälters zumindest bereichsweise entlang einer Einführrichtung aufnehmen, wobei die Handhabungseinrichtung zumindest bereichsweise hintergriffen werden kann. Insbesondere kann das Hintergreifen der Handhabungseinrichtung durch eine Verlagerung der komplementären Handhabungseinrichtung relativ zur Handhabungseinrichtung des Behälters entlang einer Heberichtung, welche senkrecht zur Einführrichtung orientiert ist, erfolgen. Durch das Hintergreifen wird die Handhabungseinrichtung gehemmt, sich entgegen der Einführrichtung zu verlagern und sich von der komplementären Handhabungseinrichtung zu lösen.

Der Behälter umfaßt eine Sicherungseinrichtung, welche den Behälter im Transportzustand hält. Mit anderen Worten wird durch die Sicherungseinrichtung ein Verlängern bzw. Strecken der Wandung des Behälters entlang der Streckrichtung gehemmt bzw. verhindert, insbesondere wenn der Behälter durch die Transportvorrichtung an der Handhabungseinrichtung ergriffen und angehoben wird, so daß die Unterseite des Behälters nicht aufliegt bzw. frei schwebt. Das Verlängern der Wandung des Behälters kann beispielsweise durch ein Entfalten bzw. Entstauchen erfolgen. Entsprechend kann die Verkürzung des Behälters entgegen der Streckrichtung durch ein Falten bzw. Stauchen der Wandung des Behälters erfolgen.
Die Sicherungseinrichtung kann im wesentlichen undehnbar ausgebildet sein. Mit anderen Worten ist die Ausdehnung der Sicherungseinrichtung entlang einer Längserstreckungsrichtung der Sicherungseinrichtung im wesentlichen konstant unabhängig davon, ob eine Kraft entlang dieser Längserstreckungsrichtung auf die Sicherungseinrichtung wirkt oder nicht. Insbesondere dehnt sich die Sicherungseinrichtung bei Anlegen einer Kraft von 1 kN, welche entlang dieser Längserstreckungsrichtung orientiert ist, um weniger als etwa 5 Prozent, bevorzugt um weniger als etwa 1 Prozent, der Länge, welche die Sicherungseinrichtung im unbelasteten Zustand aufweist. Daher kann der Behälter im Transportzustand in geringem Umfang streckbar bzw. verkürzbar sein. Beispielsweise kann die Länge des Behälters im Transportzustand entlang der Streckrichtung um eine Länge von weniger als etwa 10 cm, bevorzugt um weniger als etwa 5 cm, insbesondere um weniger als etwa 1 cm verkürzt bzw. verlängert werden.

Unbeschadet der Undehnbarkeit kann die Sicherungseinrichtung flexibel ausgebildet sein. Insbesondere kann die Sicherungseinrichtung biegsam in einer Richtung ausgebildet sein, die senkrecht zu der Längserstreckungsrichtung orientiert ist, in welcher die Sicherungseinrichtung undehnbar ausgebildet ist.

Dabei ist der Behälter derart auslegt, daß die Handhabungseinrichtung während des Hebens und Transportierens des Behälters durch die Transportvorrichtung, insbesondere beim Einsetzen des Behälters in die Haltevorrichtung, derart orientiert ist, daß die Handhabungseinrichtung an der Oberseite des Behälters angeordnet ist. Die zumindest eine Sicherungseinrichtung umfängt die Wandung des Behälters zumindest bereichsweise, bevorzugt vollständig, um eine Verlagerung der Oberseite des Behälters relativ zur Unterseite des Behälters zu hemmen. Es versteht sich, das auch eine Vielzahl von, insbesondere gleichartigen bzw. identischen, Sicherungseinrichtungen vorgesehen sein können. Beispielsweise kann der Behälter zwei, drei, vier, fünf oder mehr Sicherungseinrichtungen aufweisen.

Bevorzugt kann die zumindest eine Sicherungseinrichtung an der Handhabungseinrichtung befestigt sein. Das Gewicht der flexiblen Wandung des Behälters kann dadurch mittels der Sicherungseinrichtung an die Handhabungseinrichtung angelegt werden. Da die Handhabungseinrichtung im wesentlichen starr ausgebildet ist

Die Sicherungseinrichtung ist entsicherbar bzw. lösbar, wobei sich der Behälter bei entsicherter bzw. gelöster Sicherungseinrichtung entlang der Streckrichtung ausdehnen kann, wobei das Volumen des Behälters konstant bleiben kann, wenn der Behälter bzw. die zumindest eine Behälteröffnung fluiddicht verschlossen ist, oder sich das Volumen des Behälters vergrößert, wenn der Behälter während oder nach dem Strecken gefüllt wird bzw. wenn sich der Druck im Inneren des Behälters verringert. Das Strecken des Behälters kann insbesondere ausschließlich aufgrund der Einwirkung der Schwerkraft auf den Behälter erfolgen. Insbesondere kann die Handhabungseinrichtung des Behälters, welche sich bevorzugt an der Oberseite des Behälter befindet, durch die Transportvorrichtung an einer konstanten Position gehalten werden, während sich der Behälter entlang der Streckrichtung streckt, beispielsweise wenn die Unterseite aufgrund der Schwerkraft zum Boden verlagert wird.

Es versteht sich, daß der Behälter auch derart gedreht sein kann, daß sich die Handhabungseinrichtung an der Unterseite des Behälters befindet, also an der Seite, welche dem Boden zugewandt ist. Es versteht sich weiter, daß die Sicherungseinrichtung ein Strecken des Behälters auch im diesem Fall hemmt. Der Einfachheit halber wird in dieser Anmeldung jedoch davon ausgegangen, daß sich die Handhabungseinrichtung im Transportzustand an der Oberseite des Behälters befindet.

Vorzugsweise weist der Behälter zumindest einen Sicherungseinrichtungsbefestigungsbereich auf, mit welchem ein zugeordneter komplementärer Sicherungseinrichtungsbefestigungsbereich der Sicherungseinrichtung in Eingriff gelangbar ist, um eine Verlagerung der Sicherungseinrichtung senkrecht zur Streckrichtung zu hemmen.

Vorteilhafterweise kann durch das Eingreifen des Sicherungseinrichtungsbefestigungsbereichs mit dem zugeordneten komplementären Sicherungseinrichtungsbefestigungsbereich verhindert werden, daß sich die Sicherungseinrichtung relativ zur Wandung des Behälters derart verlagert, daß die Position der Wandung nicht mehr durch die Sicherungseinrichtung gehalten bzw. gesichert werden kann und der Behälter dadurch nicht mehr im Transportzustand verbleiben würde, sondern sich unbeabsichtigt und unkontrolliert zumindest bereichsweise strecken könnte.

Es versteht sich, daß der Behälter auch eine Vielzahl von Sicherungseinrichtungsbefestigungsbereichen aufweisen kann, beispielsweise zwei, drei, vier, fünf, sechs, sieben oder mehr Sicherungseinrichtungsbefestigungsbereiche. Dabei können der zumindest eine komplementäre Sicherungseinrichtungsbefestigungsbereich der Sicherungseinrichtung lösbar oder unlösbar mit einem zugeordneten Sicherungseinrichtungsbefestigungsbereich verbunden sein. Bevorzugt kann der zumindest eine Sicherungseinrichtungsbefestigungsbereich an der Außenseite der Wandung des Behälters ausgebildet sein. Insbesondere kann der zumindest eine Sicherungseinrichtungsbefestigungsbereich starrer ausgebildet sein, als die flexible Wandung des Behälters.

Bevorzugt ist der zumindest eine Sicherungseinrichtungsbefestigungsbereich an einer Seite der Wandung des Behälters angeordnet, welche der Handhabungseinrichtung entlang der Streckrichtung gegenüberliegt.

Vorteilhafterweise kann die Sicherungseinrichtung die Wandung des Behälters umfangen, wobei die Sicherungseinrichtung an zwei gegenüberliegenden bzw. entgegengesetzten Seiten des Behälters an der Wandung befestigt sein kann, insbesondere an der Handhabungseinrichtung und dem gegenüberliegenden Sicherungseinrichtungsbefestigungsbereich. Es versteht sich, daß eine Vielzahl von Sicherungseinrichtungsbefestigungsbereichen entlang eines Umfangs des Behälters bzw. der Wandung des Behälters angeordnet sein können. Die zumindest eine Sicherungseinrichtung kann dann eine entsprechende Anzahl von komplementären Sicherungseinrichtungsbefestigungsbereichen aufweisen und entlang des Umfangs des Behälters derart angeordnet sein, daß die Sicherungseinrichtungsbefestigungsbereiche jeweils mit einem zugeordneten komplementären Sicherungseinrichtungsbefestigungsbereich verbunden bzw. daran lösbar oder unlösbar befestigt sind. Durch eine unlösbare Verbindung der komplementären Sicherungseinrichtungsbefestigungsbereiche an den zugeordneten Sicherungseinrichtungsbefestigungsbereichen ist die Sicherungseinrichtung vorteilhafterweise unverlierbar mit dem Behälter verbunden, so daß die Sicherungseinrichtung nach dem Gebrauch des Behälters wiederverwendet werden kann, um den Behälter zu sichern, nachdem der Behälter wieder zusammengefaltet bzw. zusammengestaucht worden ist.

Bevorzugt ist der zumindest eine Sicherungseinrichtungsbefestigungsbereich an einer Bodenplatte ausgebildet, wobei die Bodenplatte bevorzugt zumindest bereichsweise eben ausgebildet ist, aber auch gekrümmt ausgebildet sein kann. Die Bodenplatte ist vorteilhafterweise starr ausgebildet und verstärkt die Wandung im Bereich des Bodens des Behälters, so daß die Wandung im Bodenbereich vorteilhafterweise vor mechanischen Beschädigungen geschützt ist, beispielsweise beim Einsetzen des Behälters in die Haltevorrichtung. Die Bodenplatte kann insbesondere lediglich bereichsweise den Boden des Behälters ausbilden. Beispielsweise kann die Bodenplatte lediglich weniger als etwa 50 Prozent oder weniger als etwa 10 Prozent des Bodens des Behälters ausbilden. Weiter bevorzugt kann die Bodenplatte den Boden des Behälters auch vollständig ausbilden. Mit anderen Worten kann die Breitenerstreckung der Bodenplatte im wesentlichen identisch zu der Breitenerstreckung des Behälters sein. Insbesondere kann der Behälter ausgelegt sein auf den Boden gestellt zu werden, wobei im Transportzustand und/oder im Anordnungszustand lediglich die Bodenplatte bzw. die Sicherungseinrichtung den Boden bzw. den Boden der Haltevorrichtung mechanisch kontaktiert, jedoch nicht die flexible Wandung.

Vorzugsweise ist die zumindest eine Sicherungseinrichtung lösbar mit der Handhabungseinrichtung verbunden. Insbesondere kann ein Lösen der Sicherungseinrichtung von der Handhabungseinrichtung ein Überführen des Behälters vom Transportzustand im den Anordnungszustand ermöglichen.

Alternativ oder zusätzlich kann die zumindest eine Sicherungseinrichtung lösbar mit der Bodenplatte verbunden sein. Insbesondere kann ein Lösen der Sicherungseinrichtung von der Bodenplatte auch ein Überführen des Behälters vom Transportzustand im den Anordnungszustand ermöglichen.

Vorzugsweise ist die Sicherungseinrichtung als flexibles, im wesentlichen nicht streckbares Band ausgebildet. Bevorzugt kann die Sicherungseinrichtung auch als flexible, im wesentlichen nicht streckbare Kette oder Seil ausgebildet sein. Das Band bzw. die Kette bzw. das Seil sind entlang der Längserstreckungsrichtung im wesentlich nicht dehnbar und senkrecht zur Erstreckungsrichtung flexibel bzw. biegbar ausgebildet. Mit anderen Worten ist die Länge der Sicherungseinrichtung im wesentlichen konstant unabhängig davon, ob eine Kraft auf die Sicherungseinrichtung wirkt oder nicht. Insbesondere dehnt sich das Band bzw. die Kette bzw. das Seil bei Anlegen einer Kraft von 1 kN um weniger als etwa 5 Prozent, bevorzugt um weniger als etwa 1 Prozent, der Länge, welche das Band bzw. die Kette bzw. das Seil im unbelasteten Zustand aufweist.

Bevorzugt ist der zumindest eine komplementäre Sicherungseinrichtungsbefestigungsbereich als Loch in dem Band ausgebildet. Entsprechend kann der zumindest eine Sicherungseinrichtungsbefestigungsbereich als Vorsprung oder Haken ausgebildet sein, welcher in das Loch eingreifen kann. Insbesondere kann das Band die Wandung des Behälter im wesentlichen vollständig umfangen, wobei an den beiden Endbereichen des Bandes jeweils ein Loch als komplementärer Sicherungseinrichtungsbefestigungsbereich ausgebildet ist. Entsprechend können zwei Sicherungseinrichtungsbefestigungsbereiche an der Handhabungseinrichtung, insbesondere an gegenüberliegenden bzw. entgegengesetzten Seiten der Handhabungseinrichtung, vorgesehen sein, beispielsweise als Haken, in welche das Band eingehängt werden kann. Weiter kann zumindest ein entsprechender Sicherungseinrichtungsbefestigungsbereich an der Bodenplatte vorgesehen sein, beispielsweise als Vorsprung, welcher in ein zugeordnetes Loch des Bandes, welches mittig im Band ausgebildet sein kann, eingreifen kann, um ein Verrutschen des Bandes zu hemmen.

Bevorzugt ist die Sicherungseinrichtung zumindest bereichsweise an der Wandung des Behälters bzw. an einem oder mehreren zusätzlichen Sicherungseinrichtungsbefestigungsbereich(en) befestigt. Beispielsweise kann die Wandung zumindest bereichsweise doppellagig ausgebildet sein, wobei zwischen beiden Lagen eine durchgängige Öffnung ausgebildet ist, so die Sicherungseinrichtung, insbesondere in Form eines Bandes, durch die durchgängige Öffnung geführt sein kann, um ein seitliches Verrutschen zu verhindern. Bevorzugt kann die Sicherungseinrichtung im Bereich der unteren Hälfte des Behälters an der Wandung des Behälters befestigt sein.

Bevorzugt bestehen das Band der Sicherungseinrichtung und die Wandung des Behälters aus identischem Material. Vorteilhafterweise können Reststücke, die bei der Herstellung der Wandung des Behälters anfallen, als Sicherungseinrichtung verwendet werden, wodurch sich weiter vorteilhafterweise eine Materialersparnis und eine Abfallreduktion ergibt. Weiter vorteilhafterweise ist es bei zulassungsbedürftigen sterilen Behältern nicht notwendig, das Zulassungsverfahren erneut durchzuführen, da das Material der Wandung für sterile Anwendungen bereits eine Zulassung besitzt.

Die Wandung des Behälters kann insbesondere aus einem mehrlagigen Material bestehen. Die Dichtigkeit der Wandung gegenüber einem Fluid, das heißt gegenüber einer Flüssigkeit, einem Gas oder einem Gemisch davon, kann durch eine fluiddichte Schicht, beispielsweise eine Innenschicht der Wandung, gewährleistet werden. Weiter können weitere Schichten bzw. Lagen vorgesehen sein, welche insbesondere dicht gegenüber Mikroorganismen sind, wie beispielsweise Einzeller, Prionen, Bakterien, Pilze, Hefen und/oder Viren.

Die Innenschicht steht in Kontakt mit dem Innenvolumen des Behälters und somit bei betriebsgemäßem Gebrauch des Behälters mit dem in dem Behälter befindlichen Fluid, beispielsweise mit Edukten bzw. Produkten einer in dem Behälter durchzuführenden bzw. durchgeführten biologischen, chemischen bzw. biochemischen Reaktion. Daher besteht die Innenschicht des Behälters bevorzugt aus einem Material, welches im Hinblick auf die durchzuführende Reaktion biologisch bzw. chemisch inert ist, das heißt, daß die Innenschicht selbst im wesentlichen nicht mit den Edukten bzw. Produkten im biologischen bzw. chemischen Sinne reagiert. Bevorzugt besteht die Innenschicht aus einem Polymer, wie beispielsweise Polyethylen (PE) und/oder Polypropylen (PP). Weiter bevorzugt ist zumindest von der Innenschicht umgebene Innere des Behälters sterilisierbar, beispielsweise mittels Ethylenoxid-Begasung, Plasmabehandlung oder Gammabestrahlung, um die Reaktion unter sterilen Bedingungen beginnen zu können. Weiter bevorzugt können die Behälteröffnungen als Sterilverbinder ausgebildet oder mit Sterilverbindern fluidisch verbunden sein, beispielsweise über einen Schlauch. Bevorzugt kann eine, insbesondere sterile, fluidische Verbindung auch über zumindest einen Schlauch herstellbar sein, welcher an den Behälter bzw. die Behälteröffnung angeschweißt ist und welcher am distalen Ende steril verschlossen sein kann. Zum Herstellen der fluidischen Verbindung kann der Schlauch mit einem externen Element, insbesondere steril, verschweißt werden, wobei während des Schweißens eine fluidische Verbindung zwischen dem Behälter und dem externen Element ausgebildet werden kann. In gebrauchsfertigem Zustand ist insbesondere zumindest das Innere des Behälters steril. Weiter bevorzugt ist der gesamte Behälter im Transportzustand vollständig sterilisiert und steril verpackt. Bevorzugt ist der Bioreaktorbehälter zum einmaligen Gebrauch bestimmt. Mit anderen Worten kann der Behälter ein Einwegprodukt sein.

Vorzugsweise ist die zumindest eine Sicherungseinrichtung als zumindest ein starres Verbindungselement ausgebildet, welches die Wandung des Behälters zumindest bereichsweise umfängt. Insbesondere kann die Sicherungseinrichtung ein Profil, ein Hohlprofil, ein Rohr oder ein Vollmaterial aus einem Metall oder einem Kunststoff umfassen. Die Sicherungseinrichtung kann dabei die Handhabungseinrichtung mit einem Sicherungseinrichtungsbefestigungsbereich starr verbinden, wobei der Sicherungseinrichtungsbefestigungsbereich bevorzugt am Boden bzw. der Bodenplatte des Behälters angeordnet sein kann. Die flexible Wandung des Behälters ist dann bevorzugt zwischen der Handhabungseinrichtung und dem Sicherungseinrichtungsbefestigungsbereich angeordnet, so daß der Behälter durch die starre Sicherungseinrichtung am Strecken entlang der Streckrichtung gehindert ist.

Bevorzugt ist das starre Verbindungselement unlösbar mit dem zumindest einen Sicherungseinrichtungsbefestigungsbereich und/oder der Bodenplatte verbunden. Vorteilhafterweise kann die Sicherungseinrichtung dadurch unverlierbar mit dem Behälter verbunden sein, so daß die Sicherungseinrichtung nach dem Gebrauch des Behälters genutzt werden kann, um den wieder gestauchten Behälter in seinem gestauchten Zustand zu halten.

Vorzugsweise ist die zumindest eine Sicherungseinrichtung wiederverwendbar ausgebildet, um den Bioreaktorbehälter nach dem Benutzen und dem Widerzusammenstauchen entgegen der Streckrichtung S im Transportzustand zu sichern.

Vorzugsweise weist die Handhabungseinrichtung eine Wellenkupplung auf, um eine innerhalb des Behälters angeordnete Welle mit einem außerhalb des Behälters angeordneten Antrieb zu verbinden. Bevorzugt umfaßt der Behälter ein Rührwerk im Inneren der flexiblen Wandung, mit welchem der Inhalt des Behälters durchmischt werden kann. Um den Behälter stauchen zu können, ist die innerhalb des Behälters angeordnete Welle zusammenschiebbar ausgeführt, beispielsweise in Form von zumindest zwei Teilwellen unterschiedlichen Durchmessers, welche teleskopartig ineinanderschiebbar sind. Insbesondere kann die Welle derart ausgeführt sein, daß sich die Welle aufgrund der Schwerkraft auseinander schiebt, wenn die Welle vertikal orientiert ist und an ihrem obere Ende gehalten ist.

Die Welle kann im Bereich der Wellenkupplung die Wandung des Behälters durchstoßen, wobei die Durchtrittsöffnung in der Wandung fluiddicht und bevorzugt steril verschlossen ist. Die Wellenkupplung ist zweckmäßigerweise im starr ausgeführten Bereich der Handhabungseinrichtung angeordnet. Die Wellenkupplung kann bevorzugt ausgelegt sein, mit einem Antrieb formschlüssig in Eingriff zu gelangen. Alternativ oder zusätzlich kann die Kraftübertragung zwischen dem Antrieb und der Wellenkupplung auch magnetisch erfolgen. In diesem Fall kann die kraftschlüssige Kopplung zwischen der Welle und dem Antrieb vorteilhafterweise auch berührungsfrei erfolgen, so daß weiter vorteilhafterweise die Welle nicht durch die Wandung des Behälter geführt werden muß, wodurch die Fluiddichtigkeit und die Sterilität des Behälter in einfacher Weise erreicht werden kann.

### Verwendung gemäß einem Aspekt

Ein Aspekt betrifft eine Verwendung einer Sicherungsvorrichtung, um einen Behälter, insbesondere einen oben beschriebenen erfindungsgemäßen Behälter oder einer bevorzugten Ausführungsform hiervon, mit einer zumindest bereichsweise flexible Wandung, welche in einem Transportzustand entgegen einer Streckrichtung zumindest bereichsweise verkürzt ist, im diesem Transportzustand zu halten, wobei die Sicherungsvorrichtung zumindest einen komplementären Sicherungseinrichtungsbefestigungsbereich aufweist, welcher mit einem zugeordneten Sicherungseinrichtungsbefestigungsbereich des Behälters in Eingriff ist. Insbesondere kann eine als Band ausgebildete Sicherungsvorrichtung verwendet werden, welche Löcher als komplementären Sicherungseinrichtungsbefestigungsbereich aufweist.

### Transportvorrichtung gemäß einem Aspekt

Ein Aspekt betrifft eine Transportvorrichtung zum Heben und Transportieren eines Behälters mit einer Handhabungseinrichtung, insbesondere eines erfindungsgemäßen Behälters oder einer bevorzugten Ausführungsform hiervon, wobei die Transportvorrichtung aufweist:
- eine komplementäre Handhabungseinrichtung, welche ausgelegt ist mit der Handhabungseinrichtung des Behälters in Eingriff zu gelangen;
- eine optionale Drehvorrichtung, durch welche die komplementäre Handhabungseinrichtung um eine horizontale Achse drehbar ist.

Ein weiterer Aspekt betrifft eine Anordnung einer erfindungsgemäßen Transportvorrichtung mit einem daran angeordneten erfindungsgemäßen Behälter.

Vorteilhafterweise kann ein Behälter mittels der Transportvorrichtung in einfacher Weise gehandhabt, transportiert und in die Haltevorrichtung eingesetzt werden.

Die komplementäre Handhabungseinrichtung ist bevorzugt zumindest bereichsweise formkongruent zur Handhabungseinrichtung des Behälters ausgebildet. Insbesondere kann die Handhabungseinrichtung des Behälters zumindest bereichsweise entlang einer Einführrichtung in eine Ausnehmung der komplementären Handhabungseinrichtung einführbar sein. Insbesondere kann die komplementäre Handhabungseinrichtung gabelförmig oder als Schelle ausgebildet sein, wobei die Handhabungseinrichtung des Behälters zwischen den beiden Armen der Gabel bzw. in die Schelle einführbar ist.

Vorzugsweise ist die Handhabungseinrichtung in der komplementären Handhabungseinrichtung arretierbar. Mit anderen Worten kann die Handhabungseinrichtung mittels einer Arretiereinrichtung gehindert werden, entgegen der Einführrichtung relativ zur der komplementären Handhabungseinrichtung verlagert zu werden. Beispielsweise kann die Arretiereinrichtung die in der komplementären Handhabungseinrichtung eingeführte Handhabungseinrichtung zumindest bereichsweise hintergreifen bzw. die Einführöffnung der komplementären Handhabungseinrichtung schließen, nachdem die Handhabungseinrichtung eingeführt ist. Die Arretiereinrichtung kann beispielsweise als schwenkbarer Bügel ausgebildet sein, welche zumindest bereichsweise um die eingeführte Handhabungseinrichtung geschwenkt wird. Der schwenkbare Bügel kann dabei nach dem Schließen der komplementären Handhabungseinrichtung durch eine Schraube, insbesondere mittels einer ohne Werkzeug drehbare Flügelschraube oder Flügelmutter, in der geschlossenen Position fixiert werden.

Alternativ oder zusätzlich kann die komplementäre Handhabungseinrichtung eine magnetische Arretiereinrichtung aufweisen, welche eine komplementäre magnetischen Arretiereinrichtung der Handhabungseinrichtung des Behälters anzieht und dadurch magnetisch fixiert, so daß der Behälter magnetisch in der komplementären Handhabungseinrichtung arretiert wird.

Weiter bevorzugt kann die komplementäre Handhabungseinrichtung in eine Auskragung, eine Ausnehmung bzw. einen Vorsprung der Handhabungseinrichtung eingreifen, so daß der Behälter mittels der Transportvorrichtung gehoben, gesenkt und/oder gedreht werden kann, ohne daß eine relative Verlagerung zwischen der komplementären Handhabungseinrichtung und der Handhabungseinrichtung erfolgt bzw. ohne daß sich der Behälter von der Transportvorrichtung löst.

Bevorzugt umfaßt die Transportvorrichtung eine motorisierte Hebevorrichtung, welche die komplementäre Handhabungseinrichtung entlang der Heberichtung, insbesondere entlang der Vertikalen, verlagern kann, insbesondere um eine Strecke von mehr als etwa einem Meter, bevorzugt um eine Strecke von mehr als etwa zwei Metern und insbesondere um eine Strecke von mehr als etwa drei Metern.

Bevorzugt umfaßt die Transportvorrichtung eine motorisierte Drehvorrichtung, durch welche die komplementäre Handhabungseinrichtung, einschließlich eines daran gehaltenen Behälters, um die horizontale Achse drehbar ist. Die horizontale Achse ist bevorzugt parallel zur Einführrichtung orientiert. Die Drehvorrichtung kann die komplementäre Handhabungseinrichtung bevorzugt um mehr als etwa 90 Grad, bevorzugt um mehr als etwa 170 Grad und insbesondere um mehr als etwa 270 Grad, drehen. Besonders bevorzugt ermöglicht die Drehvorrichtung eine oder mehrere vollständige Drehungen der komplementären Handhabungseinrichtung um die horizontale Achse.

Bevorzugt ist die Transportvorrichtung verfahrbar, insbesondere motorisiert verfahrbar. Dazu kann die Transportvorrichtung eine Vielzahl von Rädern aufweisen, wobei bevorzugt zumindest ein Rad mit einem Antrieb verbunden sein kann. Der Antrieb kann beispielsweise elektrisch erfolgen.

### Verfahren gemäß einem Aspekt

Ein Aspekt betrifft ein Verfahren zum Einsetzen eines zumindest bereichsweise flexiblen, zusammenstauchbaren Behälters, insbesondere eines erfindungsgemäßen Behälters oder einer bevorzugten Ausführungsform hiervon, in eine Haltevorrichtung, wobei der Behälter
- eine Handhabungseinrichtung,
- eine Sicherungseinrichtung, welche ein Entfalten des Behälters hemmt, aufweist und wobei das Verfahren die Schritte umfaßt:
- Bereitstellen des Behälters in einem Transportzustand, in welchem der Behälter zumindest bereichsweise entgegen einer Streckrichtung zusammengestaucht ist;
- Befestigen der Handhabungseinrichtung des Behälters an einer komplementären Handhabungseinrichtung einer Transportvorrichtung, insbesondere einer erfindungsgemäßen Transportvorrichtung oder einer bevorzugten Ausführungsform hiervon;
- Verlagern der Transportvorrichtung, wobei der Behälter in den Halter zumindest bereichsweise eingebracht wird;
- Lösen der Sicherungseinrichtung, wodurch sich der Behälter zumindest bereichsweise entlang der Streckrichtung entfaltet, um von dem Transportzustand in einen Anordnungszustand überführt zu werden; und
- Lösen der Handhabungseinrichtung von der komplementären Handhabungseinrichtung.

Vorteilhafterweise kann der Behälter in einfacher Weise in die Haltevorrichtung eingesetzt werden. Der Behälter wird im Transportzustand bereitgestellt, beispielsweise auf einer Transportpallette, welche in einem Lagerraum gelagert ist. Bevorzugt ist der Behälter steril verpackt. Weiter bevorzugt kann der Behälter mit der Handhabungseinrichtung nach unten orientiert gelagert und bereitgestellt werden, so daß die Sicherungseinrichtung nicht mechanisch belastet wird, wenn der Behälter an den Handhabungseinrichtung ergriffen und angehoben wird. Andererseits ist es auch möglich den Behälter derart orientiert zu lagern, daß sich die Handhabungseinrichtung oben befindet, wobei die Sicherungseinrichtung vorteilhafterweise verhindert, daß sich der Behälter beim Anheben, insbesondere mittels der Transportvorrichtung, entlang der Streckrichtung ausdehnt. Allerdings steht die Sicherungseinrichtung in diesem Fall nach dem Anheben unter mechanischer Belastung bzw. Spannung, da das Gewicht der flexiblen Wandung des Behälters und der optionalen ausziehbaren Welle im Inneren des Behälters auf die Sicherungseinrichtung einwirkt.

Es versteht sich, daß das Entfernen der sterilen Verpackung des Behälters vor oder nach dem Anheben des Behälters erfolgen kann. Ebenso kann das Lösen der Sicherungseinrichtung vor oder nach dem Anheben und vor oder nach dem optionalen Drehen des Behälters erfolgen.

Bevorzugt wird der Behälter im Transportzustand mittels der Transportvorrichtung von dem Lagerplatz des Behälters zur Haltevorrichtung gefahren und soweit vertikal gehoben, bis der Behälter in die Haltevorrichtung, insbesondere von oben, hineingesetzt werden kann. Dabei können der Lagerplatz und die Haltevorrichtung räumlich getrennt sein, beispielsweise durch ein Tor bzw. eine sterile Schleuse.

Alternativ kann der Behälter auch seitlich in die Haltevorrichtung eingesetzt werden, wenn die Haltevorrichtung über eine entsprechende seitlich Öffnung in der Haltevorrichtungswandung aufweist. Beispielsweise kann die Haltevorrichtung eine seitliche Türe aufweisen, durch welche der Behälter in die Haltevorrichtung einführbar ist und welche nach dem Einführen des Behälters verschlossen werden kann.

Zum Transport wird der Behälter mittels der Handhabungseinrichtung an der komplementären Handhabungseinrichtung der Transportvorrichtung befestigt. Das Befestigen kann mittels magnetischer Anziehung, durch Klemmen, durch Verschrauben, durch einen Bajonettverschluß oder auf ähnliche Weise erfolgen. Der Behälter kann dann mittels der Transportvorrichtung von dem Lagerplatz des Behälters zur Haltevorrichtung gefahren und in diese zumindest bereichsweise eingesetzt werden. Insbesondere kann die Haltevorrichtung lediglich eine obere Öffnung aufweisen, durch welche der Behälter einsetzbar ist. Daher kann der Behälter mittels der Transportvorrichtung soweit vertikal angehoben werden, daß der Behälter von oben in die Haltevorrichtung hinein geführt werden kann. Um den Behälter möglichst wenig anheben zu müssen, kann das Lösen der Sicherungseinrichtung bevorzugt dann erfolgen, wenn der Behälter bereits über der Öffnung der Haltevorrichtung angeordnet ist. Durch das Lösen der Sicherungseinrichtung streckt bzw. entfaltet sich der Behälter schwerkraftbedingt zumindest bereichsweise entlang der Streckrichtung, insbesondere entlang der Vertikalen, und damit bevorzugt in die Haltevorrichtung hinein. Optional kann der Behälter nach dem Entfalten mittels der Transportvorrichtung weiter gesenkt werden, um korrekt in der Haltevorrichtung angeordnet zu werden.

Für den bevorzugten Fall, daß der Behälter ein entlang der Streckrichtung längenvariables Element aufweist, insbesondere eine Welle eines Rührwerks, erfolgt nach dem Lösen der Sicherungseinrichtung, insbesondere lediglich aufgrund des Wirkens der Schwerkraft, das Strecken des längenvariablen Elements entlang der Streckrichtung.

Die komplementäre Handhabungseinrichtung kann dann von der Handhabungseinrichtung des Behälters gelöst werden, um die Transportvorrichtung zu entfernen.

Das Verfahren kann die zusätzlichen Schritte des Stauchens und/oder des Falten des Behälters nach dem Benutzen entgegen der Streckrichtung (S) und des Befestigens der Sicherungseinrichtung aufweisen, wodurch der Behälter wieder in den Transportzustand überführt wird. Vorteilhafterweise kann der Behälter nach dem Benutzen, beispielsweise dem Durchführen eines biotechnologischen Prozesses, in platzsparender Weise transportiert bzw. entsorgt werden. Mit anderen Worten bleibt die Sicherungseinrichtung beim Lösen intakt, so daß die Sicherungseinrichtung wiederverwendbar ist.

Die gelöste Sicherungseinrichtung kann nach dem Lösen weiter an dem Behälter befestigt sein und/oder zwischen dem Behälter und der Innenwandung der Haltevorrichtung angeordnet sein, so daß die Sicherungseinrichtung nach dem Gebrauch des Behälters vorteilhafterweise wieder bereitgestellt ist, um den Behälter zu sichern. Bevorzugt kann der Schritt des Befestigens der Sicherungseinrichtung an der Haltevorrichtung erfolgen. Insbesondere kann die Haltevorrichtung entsprechend dem Behälter zumindest einen Sicherungseinrichtungsbefestigungsbereich aufweisen, an welchem die Sicherungseinrichtung befestigbar ist.

Für den Fall, daß die Sicherungseinrichtung als Band ausgebildet ist, können an der Haltevorrichtung beispielsweise Haken oder Vorsprünge vorgesehen sein, wobei in dem Band zugeordnete Löcher ausgebildet sind, mit welchen das Band an den Haken bzw. an dem Vorsprung einhängbar bzw. befestigbar ist. Alternativ oder zusätzlich kann das Band zwischen der flexiblen Behälterwandung und der Innenwandung des Haltevorrichtung, insbesondere nach dem Befüllen des Behälters, eingeklemmt sein und über den Rand der Öffnung der Haltevorrichtung überstehen bzw. überhängen.

Für den Fall, daß die zumindest eine Sicherungseinrichtung als starre Verbindung, beispielsweise als Stange oder Rohr ausgebildet ist, kann die Sicherungseinrichtung zwischen der flexiblen Behälterwandung und der Innenwandung des Haltevorrichtung, insbesondere nach dem Befüllen des Behälters, eingeklemmt sein, wobei die starre Sicherungseinrichtung die Wandung des flexiblen Behälters bereichsweise eindrückt. Insbesondere bei Gebrauch des Behälters mit einem darin angeordneten Rührwerk, wird beim Rühren der Fließquerschnitt des in dem Behälter gerührten Fluids im Bereich der durch die Sicherungseinrichtung eingedrückten Wandung lokal verringert, so daß an diesen Stellen die Fließgeschwindigkeit lokal erhöht wird, wodurch sich vorteilhafterweise Turbulenzen ergeben, welche zu einer verbesserten Durchmischung des Fluids führen.

Nach dem Gebrauch des Behälters, der bevorzugt ein Einwegbehälter sein kann, wird der Behälter wieder mittels der Handhabungseinrichtung an der Transportvorrichtung befestigt und aus der Haltevorrichtung gehoben. Dabei kann der Behälter optional vor oder nach dem Herausheben wieder entgegen der Streckrichtung gestaucht und im gestauchten Zustand mittels der zumindest einen Sicherungseinrichtung gesichert werden.

### Figurenbeschreibung

Nachfolgend werden bevorzugte Ausführungsformen der vorliegenden Erfindung anhand der beigefügten Zeichnungen beispielhaft erläutert. Einzelne Merkmale der gezeigten bevorzugten Ausführungsformen können zu weiteren bevorzugten Ausführungsformen kombiniert werden. Es zeigen:
- Figur 1a: eine perspektivische Ansicht einer bevorzugten Ausführungsform eines Behälters im Transportzustand;
- Figur 1b: einen Schnitt durch den in Figur 1a gezeigten Behälter;
- Figur 1c: einen weiteren Schnitt durch den in Figur 1a gezeigten Behälter, welcher senkrecht zu dem in Figur 1b gezeigten Schnitt orientiert ist;
- Figur: 1d eine Draufsicht auf den in Figur 1a gezeigten Behälter;
- Figur 2a: eine perspektivische Ansicht einer Anordnung einer bevorzugten Ausführungsform einer Transportvorrichtung mit einem angehängten Behälter im Transportzustand;
- Figur 2b: eine Seitenansicht auf die in Figur 2a gezeigte Anordnung;
- Figur 2c: einen Seitenansicht auf die in Figur 2a gezeigte Anordnung, welche senkrecht zu der in Figur 2b gezeigten Seitenansicht orientiert ist;
- Figur 2d: eine Draufsicht auf die in Figur 2a gezeigte Anordnung;
- Figur 3a: eine perspektivische Ansicht einer Anordnung einer bevorzugten Ausführungsform einer Transportvorrichtung;
- Figur 3b: eine Seitenansicht auf die in Figur 3a gezeigte Transportvorrichtung;
- Figur 3c: einen Seitenansicht auf die in Figur 3a gezeigte Transportvorrichtung, welche senkrecht zu der in Figur 3b gezeigten Seitenansicht orientiert ist;
- Figur 4a: eine perspektivische Ansicht einer Anordnung einer bevorzugten Ausführungsform einer Transportvorrichtung mit einem angehängten Behälter im Anordnungszustand;
- Figur 4b: eine Seitenansicht auf die in Figur 4a gezeigte Anordnung;
- Figur 4c: eine Seitenansicht auf die in Figur 4a gezeigte Anordnung, welche senkrecht zu der in Figur 4b gezeigten Seitenansicht orientiert ist;
- Figur 4d: eine Draufsicht auf die in Figur 4a gezeigte Anordnung;
- Figur 5a: eine perspektivische Ansicht einer bevorzugten Ausführungsform eines Behälters im Anordnungszustand;
- Figur 5b: einen Schnitt durch den in Figur 5a gezeigten Behälter;
- Figur 5c: einen weiteren Schnitt durch den in Figur 5a gezeigten Behälter, welcher senkrecht zu dem in Figur 5b gezeigten Schnitt orientiert ist;
- Figur 5d: eine Draufsicht auf den in Figur 5a gezeigten Behälter;
- Figur 6a: eine Seitenansicht einer Transportvorrichtung mit einem daran befestigten Behälter;
- Figur 6b: eine Seitenansicht der Transportvorrichtung beim Einführen des Behälter in eine Haltevorrichtung;
- Figur 6c: eine Seitenansicht der Transportvorrichtung beim Strecken des Behälters in der Haltevorrichtung;
- Figur 7: eine Ausführungsform einer Sicherungseinrichtung.

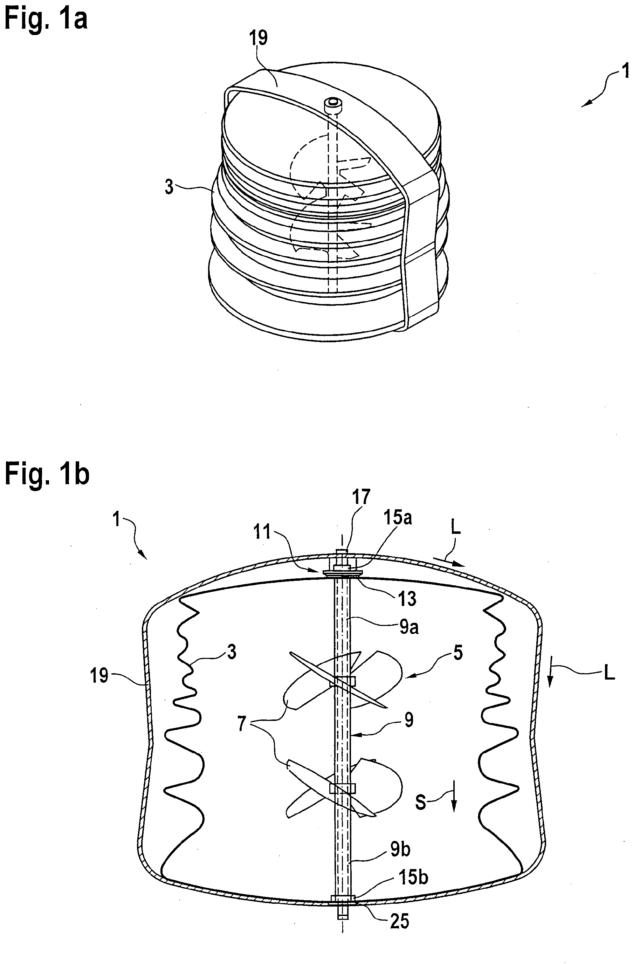

Die Figuren 1a bis 1d zeigen eine bevorzugte Ausführungsform eines Behälters 1 im Transportzustand in verschiedenen Ansichten. Der Behälter kann bevorzugt als "Bioreaktorbehälter" ausgebildet sein. Der Behälter 1 umfaßt eine zumindest bereichsweise flexible Wand bzw. Wandung 3 und ist beispielsweise zur Aufnahme, zum Mischen, zur Speicherung und zum Spenden von Fluiden, insbesondere von sterilen Fluiden bzw. Medien unter sterilen Bedingungen, ausgelegt. Die Wandung 3 umfaßt zumindest eine flexible, fluiddichte Schicht bzw. Lage, so daß durch die Wandung 3 ein variables Innenvolumen des Behälters 1 umgeben ist. Die Wandung 3 kann auch aus mehreren Schichten bzw. Lagen bestehen, welche insbesondere durch Laminieren und/oder Verkleben und/oder Verschweißen zumindest teilweise miteinander verbunden sein können. Das Innenvolumen des Behälters 1 ist über Behälteröffnungen (nicht gezeigt) mit der Umgebung oder mit weiteren Elementen, wie zum Beispiel Fluidleitungen, fluidisch verbindbar. Es versteht sich, daß die Wandung 3 insbesondere im Bereich der Behälteröffnungen versteift bzw. starr ausgebildet sein kann, damit die Behälteröffnungen formstabil sind und gegebenenfalls daran anschließende Anschlüsse und Verbinder dicht bleiben. Über die Behälteröffnungen kann der Behälter 1 befüllt und entleert werden.

Der gezeigte Behälter 1 weist eine im wesentlichen zylindrische Form auf. Es versteht sich, daß der Behälter 1 auch eine quaderförmige, eine tetraederförmige, eine kugelförmige, eine prismenförmige oder eine sonstige beliebige Gestalt aufweisen kann.

Die eine zumindest bereichsweise flexible Wandung 3 ist im Transportzustand entgegen einer Streckrichtung S zumindest bereichsweise verkürzt, insbesondere zusammengestaucht bzw. gefaltet, so daß ihre Maße entlang der Streckrichtung S reduziert sind. Der Behälter umfaßt weiter ein Rührwerk 5 im Inneren der flexiblen Wandung 3, so daß der Inhalt des Behälters mittels des Rührwerks 5 durchmischbar bzw. in Bewegung versetzbar ist. Das Rührwerk umfaßt bevorzugt ein oder mehrere (z.B. zwei) Rührquirle bzw. Flügel 7, welche an einer längenveränderbaren Welle 9 befestigt sind. Die längenveränderbaren Welle 9 ist bevorzugt als zusammenschiebbare Welle 9 ausgeführt, beispielsweise in Form von zumindest zwei Teilwellen 9a, 9b unterschiedlichen Durchmessers, welche gegeneinander verschiebbar (insbesondere teleskopartig zumindest teilweise ineinanderschiebbar) sind, um den Behälter 1 entgegen der Streckrichtung S verkürzen zu können.

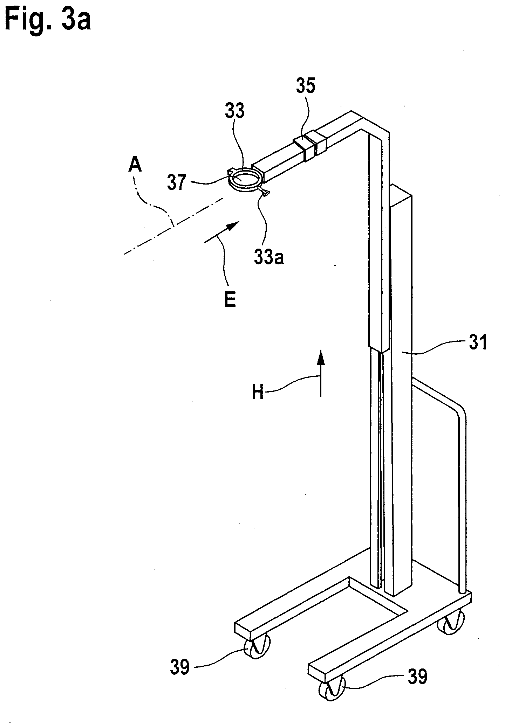

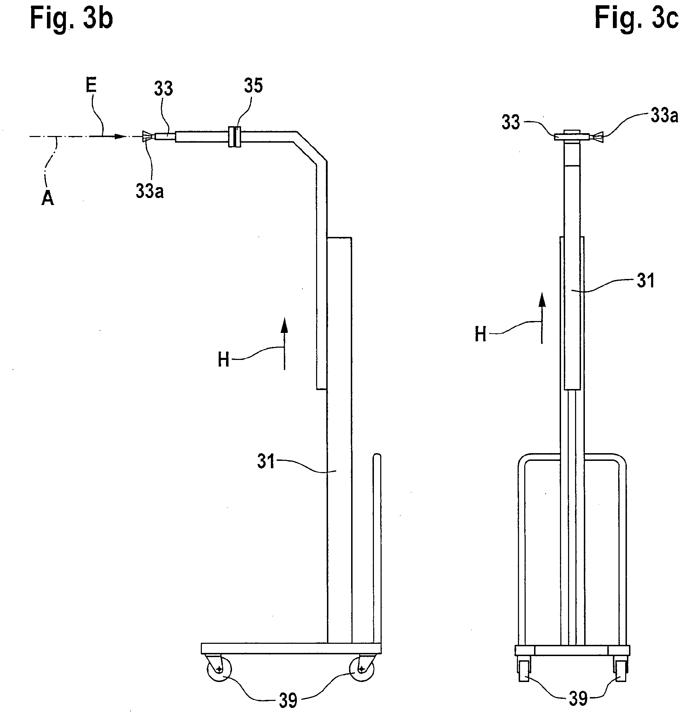

Der Behälter 1 weist weiter eine mit der Wandung 3 verbundene Handhabungseinrichtung 11 auf. Die Handhabungseinrichtung 11 ist derart ausgelegt, um mit einer komplementären Handhabungseinrichtung 33 (in den Figuren 3a bis 3c gezeigt) einer Transportvorrichtung 31 in Eingriff zu gelangen. Dadurch kann der Behälter 1 mittels der Handhabungseinrichtung 11 an einer Transportvorrichtung 31 (ebenfalls in den Figuren 3a bis 3c gezeigt) befestigt werden, um den Behälter 1 heben und/oder transportieren zu können. Dazu ist die Handhabungseinrichtung 11 insbesondere mechanisch starrer ausgebildet als die flexible Wandung 3 des Behälters 1. Insbesondere ist die Handhabungseinrichtung 11 derart starr und unverformbar ausgebildet, daß die komplementäre Handhabungseinrichtung 33 der Transportvorrichtung 31 den Behälter 1 an der Handhabungseinrichtung 11 greifen bzw. aufnehmen kann, ohne die flexible Wandung 3 des Behälters 1 zu verformen.

In der gezeigten bevorzugten Ausführungsform ist die Handhabungseinrichtung 11 an einer Durchtrittsöffnung 13 für die Welle 9 angeordnet, wobei die Durchtrittsöffnung 13 in der Wandung 3 fluiddicht und bevorzugt steril verschlossen ist. Weiter ist ein Wellenlager 15a in bzw. an der Durchtrittsöffnung 13 angeordnet, in welchem die Welle 9 drehbar gelagert ist.

Oberhalb des Wellenlagers 15a und außerhalb des von der Wandung 3 eingeschlossenen Innenvolumens des Behälters 1 ist eine Wellenkupplung 17 mit der Welle 9 verbunden, welche mit einem Antrieb (nicht gezeigt) formschlüssig und/oder kraftschlüssig in Eingriff gelangen kann, um das Rührwerk 5 anzutreiben. Als Alternative zu der in den Figuren 1a bis 1d gezeigten Wellenkupplung 17 kann auch eine magnetische Kraftübertragung zwischen dem Antrieb und der Wellenkupplung erfolgen. In diesem Fall kann die Wellenkupplung vorteilhafterweise innerhalb des von der Wandung 3 eingeschlossenen Innenvolumens des Behälters 1 angeordnet sein, wodurch vorteilhafterweise eine aufwendige Abdichtung der durch die Wandung tretenden Wellen entfallen kann, da die Welle vollständig im Innenvolumen angeordnet ist. Die Kopplung zwischen der Welle und dem Antrieb erfolgt insbesondere im wesentlichen berührungsfrei.

Der Behälter 1 weist ferner zumindest eine Sicherungseinrichtung 19 auf, welche im Transportzustand ein Entfalten der Wandung 3 des Behälters 1 entlang der Streckrichtung S zumindest teilweise oder bereichsweise verhindert. Die zumindest eine Sicherungseinrichtung 19 ist bevorzugt lösbar an der Handhabungseinrichtung 11 befestigt, so daß ein Lösen der Sicherungseinrichtung 19 von der Handhabungseinrichtung 11 ein Überführen des Behälters vom Transportzustand in den Anordnungszustand ermöglicht.

In der gezeigten bevorzugten Ausführungsform ist genau eine Sicherungseinrichtung 19 vorgesehen, welche als flexibles, im wesentlichen nicht streckbares Band 19 ausgebildet ist. Es versteht sich jedoch, daß auch zwei oder mehr Sicherungseinrichtungen 19 bzw. Bänder 19 vorgesehen sein können. Das zumindest eine Band 19 umfängt die Wandung 3 des Behälters 1 zumindest bereichsweise, bevorzugt vollständig, entlang eines Umfangs des Behälters 1, um eine Verlagerung der Oberseite des Behälters 1 relativ zur Unterseite des Behälters 1, also ein Strecken des Behälters entlang der Streckrichtung S zu hemmen bzw. diesem entgegenzuwirken. Wird der Behälter an der Handhabungseinrichtung 11 ergriffen und vertikal gehoben, so wirkt die Gewichtskraft der flexiblen Wandung 3 und/oder des Rührwerks 5 auf das Band 19. Das Band 19 ist entlang der Längserstreckungsrichtung L des Bandes 19 im wesentlich nicht dehnbar und senkrecht zur Längserstreckungsrichtung L flexibel bzw. biegbar ausgebildet. Somit ist die Länge des Bandes 19 unabhängig von einer mechanischen Belastung, also unabhängig davon ob der Behälter 1 an der Handhabungseinrichtung 1 angehoben wird oder nicht, bevorzugt im wesentlichen konstant. Dadurch kann der Behälter 1 im gestauchten Zustand gehoben werden.

Dadurch erlaubt das zumindest eine Band 19 ein Heben und einen Transport des Behälters 1 im verkürzten Transportzustand.

Bevorzugt bestehen das zumindest eine Band 19, welches die Sicherungseinrichtung 19 ausbildet, und die Wandung 3 des Behälters 1 aus identischem Material. Vorteilhafterweise können dadurch Reststücke, die bei der Herstellung der Wandung 3 des Behälters 1 anfallen, als Sicherungseinrichtung verwendet werden. Als weiterer Vorteil ist nicht notwendig einen bereits als sterilen Behälter bzw. als Bioreaktorbehälter zugelassenen Behälter 1, bei Verwendung der bevorzugten Sicherungseinrichtung erneut zuzulassen, da das Material der Wandung und damit das Material der Sicherungseinrichtung in diesem Fall für sterile Anwendungen bereits eine Zulassung besitzt.

Der Behälter 1 kann bevorzugt ein Innenvolumen von mehr als etwa 1000 Litern, weiter bevorzugt von mehr als 3000 Litern, insbesondere von mehr als etwa 5000 Litern aufweisen. Beispielsweise sind bereits Behälter 1 mit einem Innenvolumen von etwa 6000 Litern technisch realisierbar. Dabei kann der Behälter 1 mit einem Rührwerk 5 versehen sein (solche Behälter 1 sind auch als "mixing bag" bekannt). Alternativ oder zusätzlich kann der Behälter 1 zumindest teilweise auch mit einem pulverförmigen bzw. rieselfähigen Feststoff gefüllt werden bzw. im Transportzustand bereit gefüllt sein (solche Behälter 1 sind auch als "mixing bag" bekannt).
Der Behälter 1 kann zumindest einen, bevorzugt zwei, drei oder mehrere, Sicherungseinrichtungsbefestigungsbereich(e) 21 aufweisen, mit welchem ein zugeordneter komplementärer Sicherungseinrichtungsbefestigungsbereich 23 der Sicherungseinrichtung 19 bzw. des Bandes 19 in Eingriff gelangbar ist, um eine Verlagerung des Bandes 19 senkrecht zur Längsrichtung L des Bandes 19 zu hemmen. Dadurch wird weiter verhindert, daß sich die Sicherungseinrichtung 19 bzw. das Band 19 relativ zur Wandung 3 des Behälters 1 derart verlagert, daß die Position der Wandung 3 nicht mehr durch die Sicherungseinrichtung bzw. das Band 19 gehalten bzw. gesichert werden kann und der Behälter 1 sich unkontrolliert streckt.

Das Band 19 ist in der gezeigten bevorzugten Ausführungsform mit zumindest einem Loch 23 als bevorzugtem komplementären Sicherungseinrichtungsbefestigungsbereich 23 versehen, wobei sich die Handhabungseinrichtung 11 und/oder die Welle 9 zumindest bereichsweise durch dieses Loch 23 hindurch erstrecken. Die Handhabungseinrichtung 11 kann dabei gleichzeitig als Sicherungseinrichtungsbefestigungsbereich 21 fungieren, um das Band an dieser Stelle des Behälters 1 zu fixieren.

Zumindest ein weiterer Sicherungseinrichtungsbefestigungsbereich 21 ist an einer Seite bzw. einem seitlichen Bereich der Wandung 3 des Behälters 1 angeordnet, welche der Handhabungseinrichtung 11 entlang der Streckrichtung S gegenüberliegt. Die Wandung 3 ist an dieser Stelle mit einer Bodenplatte 25 versehen, welche bereichsweise den Boden des Behälters 1 ausbildet und neben dem Sicherungseinrichtungsbefestigungsbereich 21 ein weiteres Wellenlager 15b aufweist, durch welches bzw. an welcher die Welle 9 drehbar gelagert ist. Die Bodenplatte 25 ist im Vergleich zur flexiblen Wandung 3 relativ starr ausgebildet. Der dem zumindest einen Sicherungseinrichtungsbefestigungsbereich 21 der Bodenplatte 25 zugeordnet komplementäre Sicherungseinrichtungsbefestigungsbereich 23 des Bandes 19 kann ebenfalls als Loch 23 in diesem Band 19 ausgebildet sein. Das Band 19 kann durchgängig bzw. geschlossen ausgebildet sein, so daß es zum Entsichern und Strecken des Behälters durchgeschnitten werden muß.

Bevorzugt weist das Band 19 zwei Enden auf, wobei in jedem Endbereich des Bandes 19 jeweils ein komplementärer Sicherungseinrichtungsbefestigungsbereich 23 bzw. ein Loch 23 ausgebildet ist. Entsprechend können an der Bodenseite des Behälters 1 bzw. an der Bodenplatte 25 ein oder zwei Sicherungseinrichtungsbefestigungsbereich(e) 21, beispielsweise ein oder zwei Vorsprünge oder Haken oder ein vorstehender Bereich des Wellenlagers 15b, vorgesehen sein, so daß die Endbereiche des Bandes 19 mit den Löchern 23 gemeinsam an einem Sicherungseinrichtungsbefestigungsbereich 21 oder jeweils an einem zugeordneten Sicherungseinrichtungsbefestigungsbereich 21 befestigt sein können.

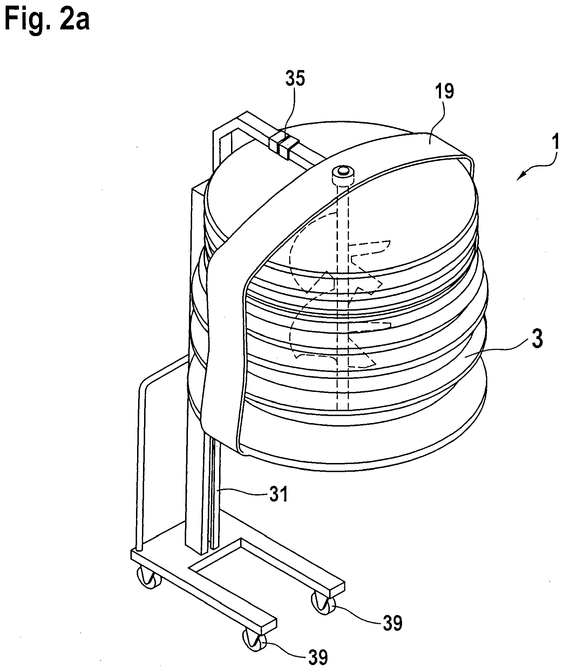

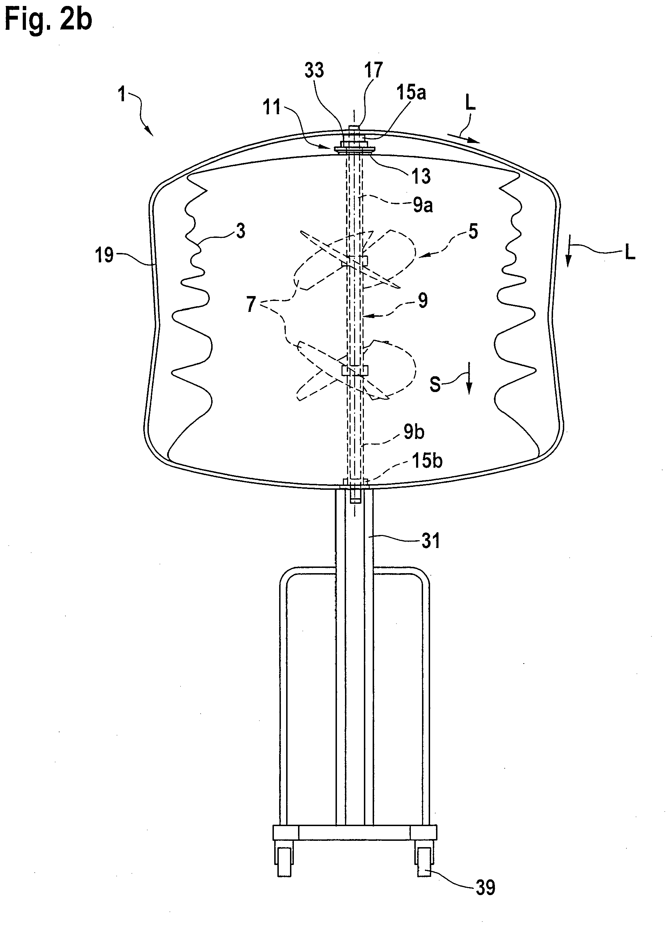

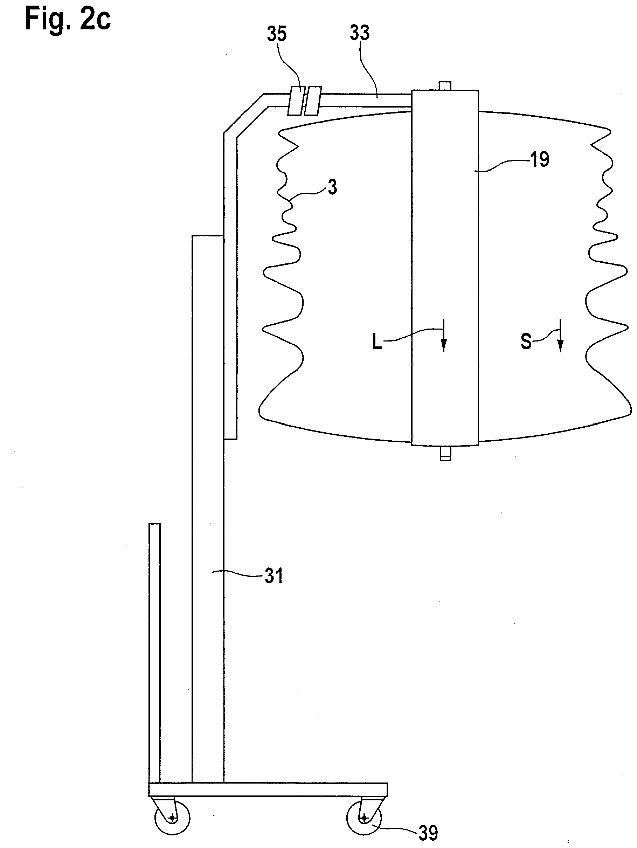

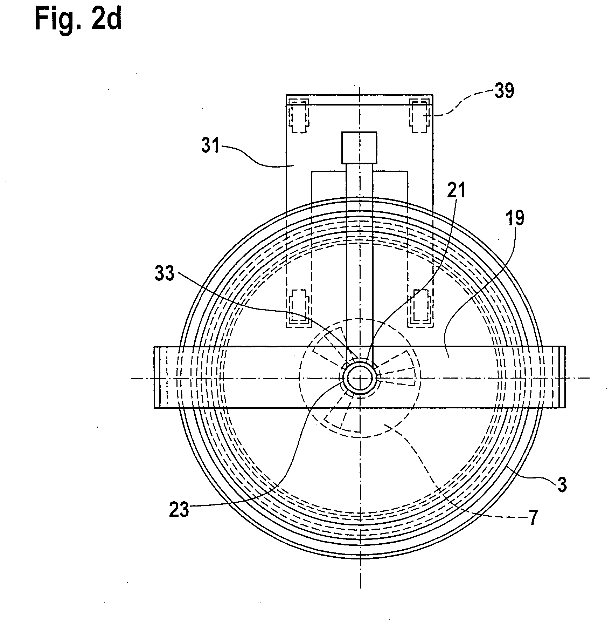

Die **Figuren 2a bis 2d** zeigen die bevorzugte Ausführungsform einer Transportvorrichtung 31 mit einem daran befestigten Behälter 1 im Transportzustand in verschiedenen Ansichten. Die Transportvorrichtung 31 weist eine komplementäre Handhabungseinrichtung 33 auf, welche ausgelegt ist mit der Handhabungseinrichtung 11 des Behälters in Eingriff zu gelangen. Die Transportvorrichtung kann weiter eine Drehvorrichtung 35 aufweisen, durch welche die komplementäre Handhabungseinrichtung 31 um eine horizontale Achse drehbar ist. Die Drehvorrichtung 35 ermöglicht es den Behälter 1 in einem gedrehten Zustand, insbesondere kopfüber, also mit der Handhabungseinrichtung 11 zum Boden gewandt, zu lagern bzw. zu transportieren und nach dem Aufnehmen des Behälters diesen mittels der Drehvorrichtung 35 umzudrehen. Die Drehvorrichtung 35 ist nicht notwendig, wenn der Behälter bereits in der richtigen Orientierung gelagert wird.

Die **Figuren 3a bis 3c** zeigen die Transportvorrichtung 31 ohne Behälter in verschiedenen Ansichten. Die komplementäre Handhabungseinrichtung 33 ist bevorzugt zumindest bereichsweise formkongruent zur Handhabungseinrichtung 11 des Behälters 1 ausgebildet. Dabei kann die komplementäre Handhabungseinrichtung 33 eine Aufnahme 37 bzw. eine Ausnehmung 37 aufweisen, in welche die Handhabungseinrichtung 11 des Behälters 1 zumindest bereichsweise entlang einer Einführrichtung E einführbar ist.

Insbesondere kann die komplementäre Handhabungseinrichtung 33 in Form einer Schelle 33 ausgebildet sein, wobei die Handhabungseinrichtung 11 des Behälters 1 in die offene Schelle 33 einführbar ist, welche nach dem Einführen der Handhabungseinrichtung 11 geschlossen und verriegelt bzw. arretiert werden kann, wobei das Verriegeln bzw. Arretieren durch eine Arretiereinrichtung 33a, beispielsweise durch eine Schraube 33a erfolgen kann, welche den schwenkbaren Teil der Schelle 33 sichert.

Bevorzugt umfaßt die Transportvorrichtung 31 eine motorisierte Hebevorrichtung, welche die komplementäre Handhabungseinrichtung 33 entlang der Heberichtung H, insbesondere entlang der Vertikalen, verlagern kann. Die Hebevorrichtung kann beispielsweise elektrisch über eine Kette oder eine Spindel und/oder hydraulisch und/oder pneumatisch angetrieben werden. Die Hebevorrichtung kann alternativ auch manuell angetrieben werden.

Weiter bevorzugt verfügt die Transportvorrichtung 31 über eine motorisierte Drehvorrichtung 35, durch welche die komplementäre Handhabungseinrichtung, bevorzugt einschließlich eines daran gehaltenen Behälters 1, um die horizontale Achse A gedreht werden kann. Alternativ kann die Drehung auch manuell erfolgen.

Die Transportvorrichtung 31 ist mit Rädern 39 versehen und dadurch verfahrbar, insbesondere motorisiert verfahrbar. Dazu kann die Transportvorrichtung 31 einen Antrieb zumindest eines Rades 39 aufweisen. Der Antrieb kann beispielsweise elektrisch erfolgen. Alternativ bzw. zusätzlich kann die Transportvorrichtung 31 auch manuell geschoben werden.

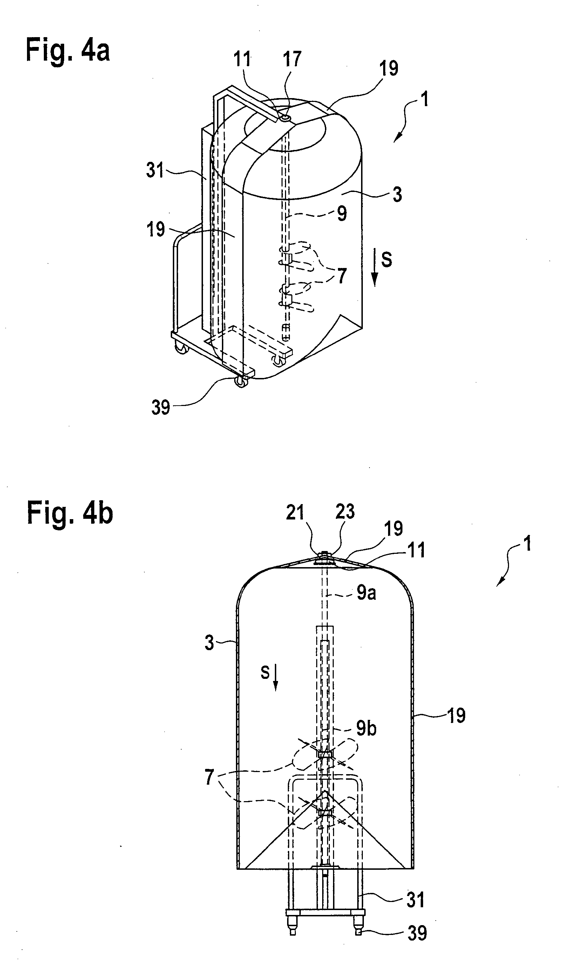

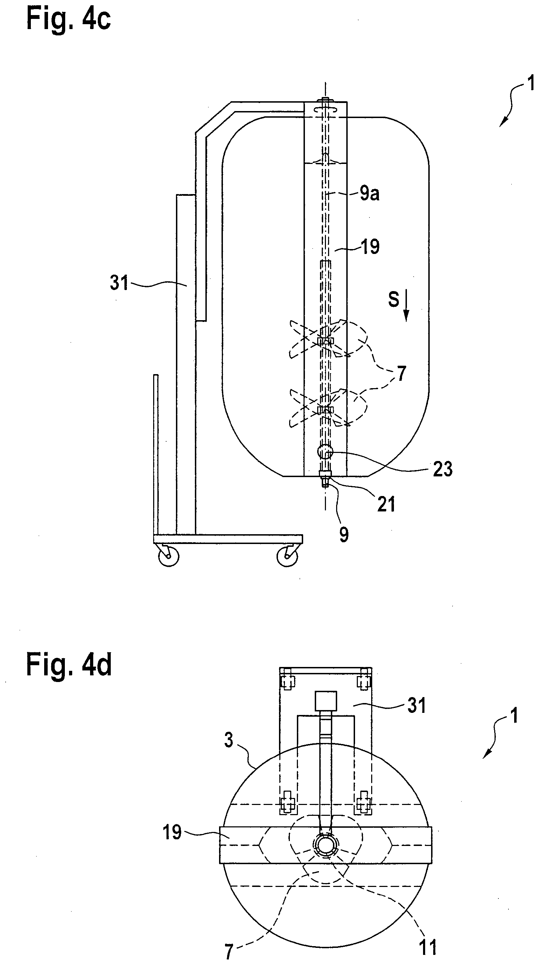

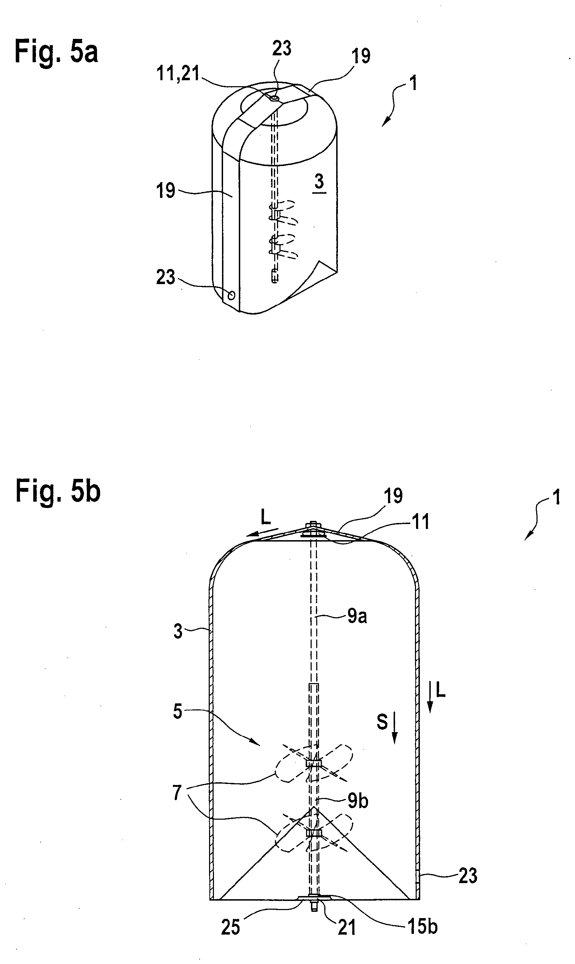

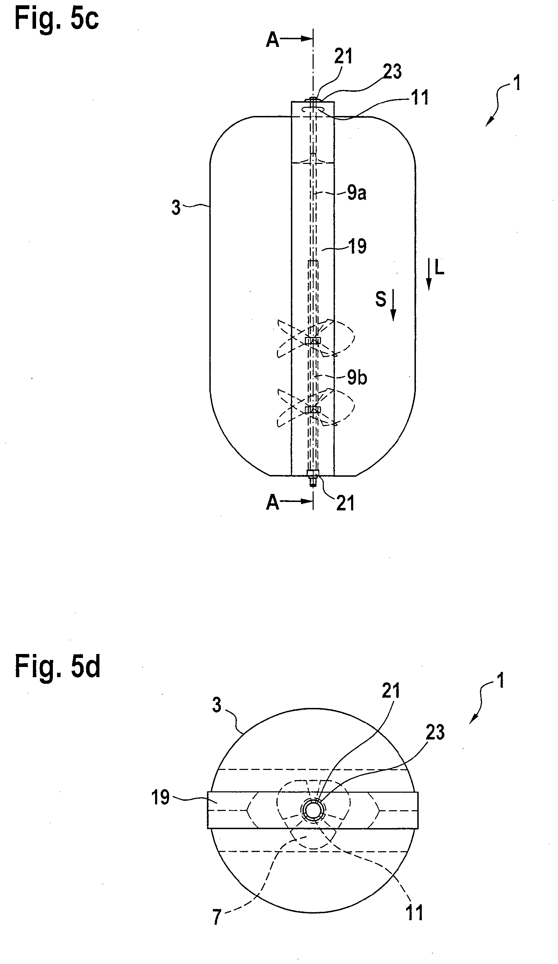

Die **Figuren 4a bis 4d** zeigen die Transportvorrichtung 31 mit einem daran befestigten Behälter 1 im gestreckten Anordnungszustand in verschiedenen Ansichten. Die **Figuren 5a bis 5d** zeigen den Behälter 1 im Anordnungszustand in verschiedenen Ansichten. Durch das Lösen der Sicherungseinrichtung 19 bzw. des Bandes 19, beispielsweise durch das Lösen bzw. Aushängen des Bandes 19 von den Sicherungseinrichtungsbefestigungsbereichen 21 an der Bodenplatte 25 streckt sich der Behälter 1 durch die Schwerkrafteinwirkung zumindest bereichsweise entlang der Streckrichtung S, welche bevorzugt parallel zur Vertikalen orientiert ist.

In der gezeigten Ausführungsform ist die Sicherungseinrichtung 19 bzw. das Band 19 noch an der Handhabungseinrichtung 11 befestigt, so daß es unverlierbar mit dem Behälter 1 verbunden ist und nach dem Gebrauch des Behälters 1 wieder verwendet werden kann. Das Strecken des Behälter 1, das heißt der Übergang des Behälter von dem Inititalzustand in den Anordnungszustand kann außerhalb oder innerhalb einer Haltevorrichtung (nicht gezeigt) für den Behälter 1 durchgeführt werden. Während des Gebrauchs befindet sich der Behälter im wesentlichen in dem in den Figuren 5a bis 5d gezeigten Zustand, wobei der Behälter während des Gebrauchs in der Regel mit einem Fluid gefüllt ist.

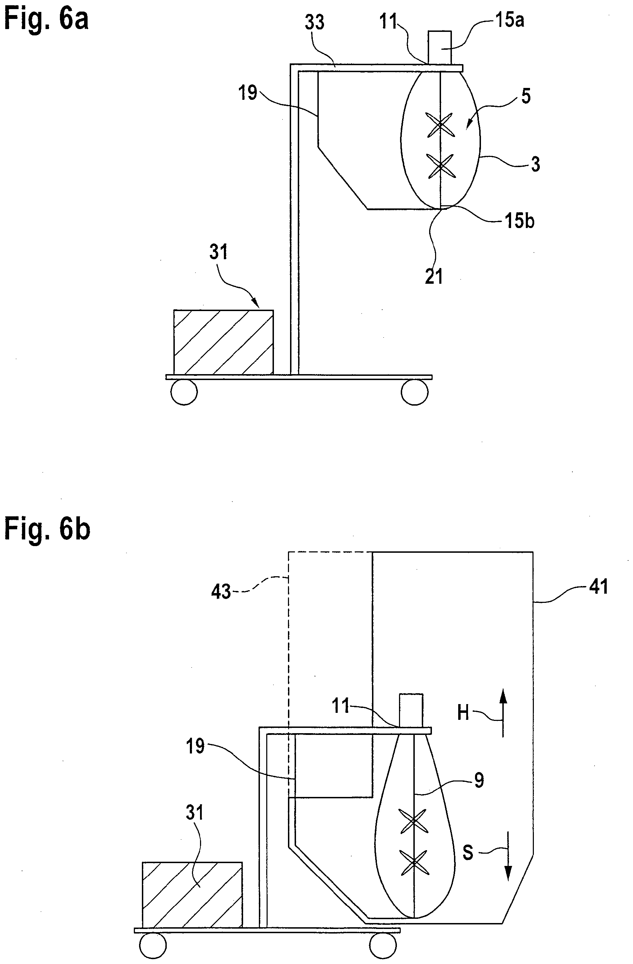

Die **Figuren 6a bis 6c** zeigen die Transportvorrichtung 31 mit einem daran befestigten Behälter 1 beim Einsetzen des Behälters in eine Haltevorrichtung 41.

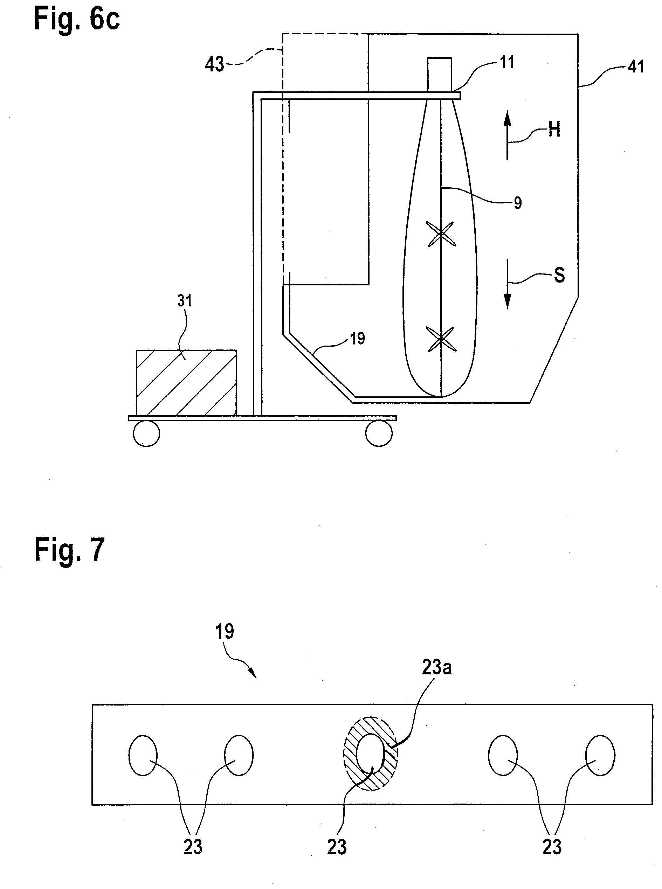

Wie in Figur 6a gezeigt wird der Behälter 1 im Transportzustand mittels der Transportvorrichtung 31 von einem Lagerplatz des Behälters 1 zur Haltevorrichtung 41 (in den Figuren 6b und 6c gezeigt) gefahren und soweit entlang der Heberichtung H gehoben, bis der Behälter 1 in die Haltevorrichtung 41 hineingesetzt werden kann. Der Behälter wird durch eine starre Sicherungseinrichtung 19 im Transportzustand gesichert, wobei die Sicherungseinrichtung eine starre Verbindung zwischen einem am Boden des Behälters 1 angeordneten Sicherungseinrichtungsbefestigungsbereich 21 und der komplementären Handhabungseinrichtung 33 der Transportvorrichtung 31 ausbildet.

Wie in der Figur 6b gezeigt kann der Behälter 1 seitlich in die Haltevorrichtung 41 eingesetzt werden. Dazu weist die Haltevorrichtung 41 eine, insbesondere verschließbare, seitliche Öffnung 43 in der Wandung der Haltevorrichtung 41 auf. Die Wandung der Haltevorrichtung 41 kann beispielsweise mittels einer seitlichen Türe verschließbar sein, durch welche der Behälter 1 im geöffneten Zustand der Türe in die Haltevorrichtung 41 einführbar ist und welche nach dem Einführen des Behälters 1 verschlossen werden kann.

Der Behälter 1 kann nach dem Einführen in die Haltevorrichtung 41 entgegen der Heberichtung H innerhalb der Haltevorrichtung 41 verlagert werden, bis der Behälter 1 auf dem Boden der Haltevorrichtung 41 aufsetzt. Dabei wird die starre Sicherungseinrichtung 19 mit in die Haltevorrichtung 41 eingeführt und zwischen dem Behälter 1 und der Innenwandung der Haltevorrichtung 41 angeordnet, so daß die Sicherungseinrichtung 19 nach dem Gebrauch des Behälters 1 vorteilhafterweise wieder bereitgestellt ist, um den Behälter 1 zu sichern.

Wie in Figur 6c gezeigt kann das Lösen der Sicherungseinrichtung 19 von der komplementären Handhabungseinrichtung 33 der Transportvorrichtung 31 dann erfolgen, wenn der Behälter bereits in der Haltevorrichtung angeordnet ist. Durch das Lösen der Sicherungseinrichtung 19 kann der Behälter 1 entlang der Streckrichtung S verlängert bzw. gestreckt werden. Die in den Figuren 6a bis 6c gezeigte Ausführungsform des Behälters 1 weist ein entlang der Streckrichtung S längenvariables Rührwerk 5 auf, wobei sich die Welle 9 des Rührwerks teleskopartig ausgebildet und damit längenvariabel ist.

Nach dem Anordnen und Strecken des Behälters 1 in der Haltevorrichtung 41 kann die komplementäre Handhabungseinrichtung 33 von der Handhabungseinrichtung 11 des Behälters 1 gelöst werden, um die Transportvorrichtung 31 zu entfernen.

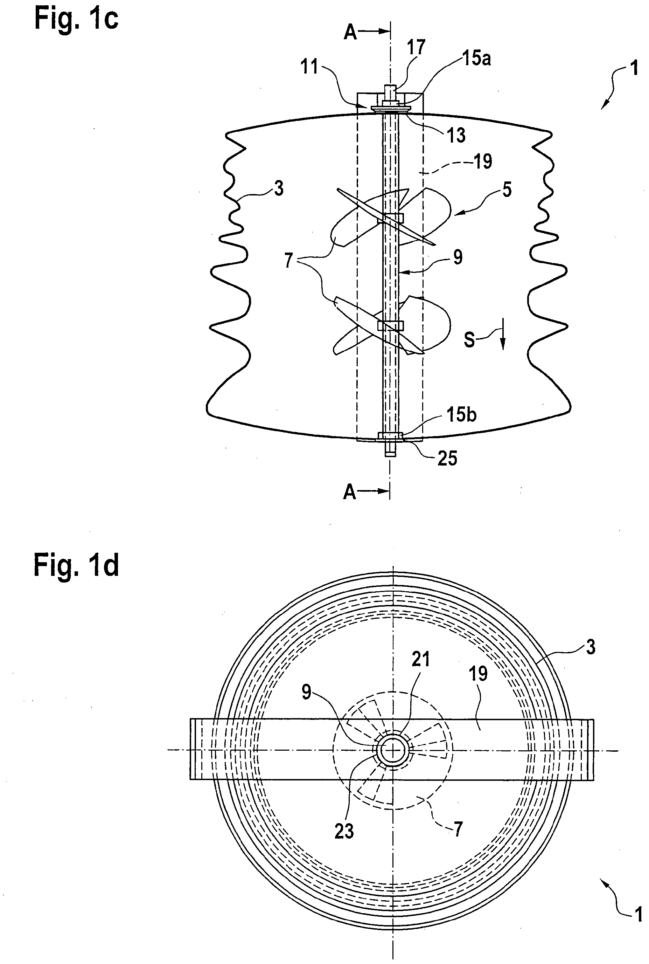

Die gelöste Sicherungseinrichtung 19 verbleibt weiter an dem Behälter 1 und ist zwischen dem Behälter 1 und der Innenwandung der Haltevorrichtung 41 angeordnet. Die starre Sicherungseinrichtung 19 kann durch ein Profil, ein Hohlprofil, ein Rohr oder ein Vollmaterial aus einem Metall oder einem Kunststoff ausgebildet sein. Um die Sicherungseinrichtung 19 an dem Behälter 1 zu befestigen, kann ein komplementärer Sicherungseinrichtungsbefestigungsbereich 23 (siehe auch Figuren 1, 2, 4 und 5) der Sicherungseinrichtung 19 mit dem Sicherungseinrichtungsbefestigungsbereich 21 des Behälter, der bevorzugt am Boden bzw. der Bodenplatte des Behälters angeordnet sein kann, befestigt sein. Dadurch kann die Sicherungseinrichtung 19 unverlierbar mit dem Behälter 1 verbunden sein.

Da die zumindest eine Sicherungseinrichtung 19 starre ausgebildet und zwischen der flexiblen Behälterwandung 3 und der Innenwandung des Haltevorrichtung 41 angeordnet ist, kann die starre Sicherungseinrichtung 19 die Wandung 3 des flexiblen Behälters 1 zumindest bereichsweise eindrücken. Beim Gebrauch des Behälters 1 und des darin angeordneten Rührwerks 5, wird beim Rühren der Fließquerschnitt des in dem Behälter 1 gerührten Fluids im Bereich der durch die Sicherungseinrichtung 19 eingedrückten Wandung 3 lokal verringert, so daß an diesen Stellen die Fließgeschwindigkeit lokal erhöht wird, wodurch sich vorteilhafterweise Turbulenzen ergeben, welche zu einer verbesserten Durchmischung des Fluids führen.

Die **Figur 7** zeigt eine bevorzugte Ausführungsform einer Sicherungseinrichtung 19, welche als flexibles Band ausgebildet ist. Die Sicherungseinrichtung 19 weist eine Vielzahl von komplementären Sicherungseinrichtungsbefestigungsbereichen 23 auf. Ein komplementärer Sicherungseinrichtungsbefestigungsbereich 23, welcher ausgelegt ist, mit einem Sicherungseinrichtungsbefestigungsbereich 21 an einem Boden bzw. an einer Bodenplatte 15 des Behälters 1 in Eingriff zu gelangen (siehe Figuren 1, 2, 4 und 5) kann vorzugsweise eine Verstärkung 23a aufweisen. Die Verstärkung 23a kann beispielsweise durch eine Materialverdickung oder durch zumindest eine zusätzliche Lage des Materials des Bandes 19 ausgebildet sein. Vorteilhafterweise kann durch die Verstärkung 23a ein mechanisch besonders beanspruchbarer komplementärer Sicherungseinrichtungsbefestigungsbereich 23 ausgebildet werden. Die weiteren komplementären Sicherungseinrichtungsbefestigungsbereiche 23 können ausgelegt sein, mit zugeordneten Sicherungseinrichtungsbefestigungsbereichen 21 an der Handhabungseinrichtung 11 des Behälters oder an der Wandung des Haltevorrichtung 41 in Eingriff zu gelangen.

Es versteht sich , daß die Sicherungseinrichtung 19 anstatt als Band alternativ als Seil,insbesondere mit zumindest einer Schlaufe als komplementärer Sicherungseinrichtungsbefestigungsbereich 23, oder als Gliederkette, wobei die in den Kettenglieder ausgebildeten Öffnungen als komplementärer Sicherungseinrichtungsbefestigungsbereich 23 dienen können, ausgebildet sein kann.

### Bezugszeichenliste

- 1: Behälter
- 3: Wandung
- 5: Rührwerk
- 7: Rührquirl
- 9: Welle
- 9a, 9b: Teilwellen
- 11: Handhabungseinrichtung
- 13: Durchtrittsöffnung
- 15a: Wellenlager
- 15b: Wellenlager
- 17: Wellenkupplung
- 19: Sicherungseinrichtung, Band
- 21: Sicherungseinrichtungsbefestigungsbereich
- 23: komplementärer Sicherungseinrichtungsbefestigungsbereich, Loch
- 23a: Verstärkung
- 25: Bodenplatte
- 31: Transportvorrichtung
- 33: komplementäre Handhabungseinrichtung, Schelle
- 33a: Arretiereinrrichtung, Schraube
- 35: Drehvorrichtung
- 37: Aufnahme
- 39: Rad
- 41: Haltevorrichtung
- 43: Öffnung in der Haltevorrichtung 41
- A: Achse
- E: Einführrichtung
- H: Heberichtung
- L: Längsrichtung des Bandes 19
- S: Streckrichtung des Behälters 1

## Patentansprüche

1. Bioreaktorbehälter (1) zum Einsetzen in eine Haltevorrichtung umfassend:
- eine zumindest bereichsweise flexible Wandung (3), welche in einem Transportzustand entgegen einer Streckrichtung (S) zumindest bereichsweise verkürzt ist; und
- eine mit der Wandung (3) verbundene Handhabungseinrichtung (11), welche ausgelegt ist, mit einer komplementären Handhabungseinrichtung (33) einer Transportvorrichtung (31) in Eingriff zu gelangen;
**dadurch gekennzeichnet, dass** die Wandung (3) in einem Transportzustand entgegen einer Streckrichtung (S) zumindest bereichsweise verkürzt ist; und
- dass eine Sicherungseinrichtung (19) vorgesehen ist, welche in einem Transportzustand ein Strecken der Wandung (3) des Bioreaktorbehälters (1) entlang der Streckrichtung (S) hemmt, wobei der Behälter (1) zumindest bereichsweise entlang der Streckrichtung (S) verlängerbar ist, wenn die Sicherungseinrichtung (19) gelöst ist.

2. Bioreaktorbehälter (1) gemäß Anspruch 1, wobei der Bioreaktorbehälter (1) zumindest einen Sicherungseinrichtungsbefestigungsbereich (21) aufweist, mit welchem ein zugeordneter komplementärer Sicherungseinrichtungsbefestigungsbereich (23) der Sicherungseinrichtung (19) in Eingriff gelangbar ist, um eine Verlagerung der Sicherungseinrichtung (19) in einer von der Streckrichtung (S) verschiedenen Richtung zu hemmen.

3. Bioreaktorbehälter (1) gemäß Anspruch 2, wobei der zumindest eine Sicherungseinrichtungsbefestigungsbereich (21) an einer Seite der Wandung (3) des Bioreaktorbehälters (1) angeordnet ist, welche der Handhabungseinrichtung (11) entlang der Streckrichtung (S) gegenüberliegt und
wobei der zumindest eine Sicherungseinrichtungsbefestigungsbereich (21) bevorzugt an einer Bodenplatte (25) ausgebildet ist.

4. Bioreaktorbehälter (1) gemäß einem der Ansprüche 1 bis 3, wobei die Sicherungseinrichtung (19) als flexibles, im wesentlichen nicht streckbares Band (19) ausgebildet ist und
wobei bevorzugt der zumindest eine komplementäre Sicherungseinrichtungsbefestigungsbereich (23) als Loch (23) in dem Band (19) ausgebildet ist.

5. Bioreaktorbehälter (1) gemäß Anspruch 4, wobei das Band (19) und die Wandung (3) des Bioreaktorbehälters (1) aus identischem Material bestehen.

6. Bioreaktorbehälter (1) gemäß einem der Ansprüche 1 bis 5, wobei die Sicherungseinrichtung (19) zumindest bereichsweise an der Wandung (3) befestigt ist.

7. Bioreaktorbehälter (1) gemäß einem der Ansprüche 1 bis 3, wobei die Sicherungseinrichtung (19) als zumindest ein starres Verbindungselement ausgebildet ist, welches die Wandung (3) des Bioreaktorbehälters (1) zumindest bereichsweise umfängt und
wobei das starre Verbindungselement bevorzugt unlösbar mit dem zumindest einen Sicherungseinrichtungsbefestigungsbereich (21) und/oder der Bodenplatte (25) verbunden ist.

8. Bioreaktorbehälter (1) gemäß einem der Ansprüche 1 bis 7, wobei die zumindest eine Sicherungseinrichtung (19) wiederverwendbar ausgebildet ist, um den Bioreaktorbehälter (1) nach dem Benutzen und dem Widerzusammenstauchen entgegen der Streckrichtung (S) im Transportzustand zu sichern.

9. Bioreaktorbehälter gemäß einem der Ansprüche 1 bis 8, wobei die Handhabungseinrichtung (11) eine Wellenkupplung (17) aufweist, um eine innerhalb des Bioreaktorbehälters (1) angeordnete Welle (9) mit einem außerhalb des Bioreaktorbehälters (1) angeordneten Antrieb zu verbinden.

10. Verwendung einer Sicherungsvorrichtung (19), um einen Bioreaktorbehälter (1) mit einer zumindest bereichsweise flexible Wandung (3), welche in einem Transportzustand entgegen einer Streckrichtung (S) zumindest bereichsweise verkürzt ist, im diesem Transportzustand zu halten,
wobei die Sicherungsvorrichtung (19) zumindest einen komplementären Sicherungseinrichtungsbefestigungsbereich (23) aufweist, welcher mit einem zugeordneten Sicherungseinrichtungsbefestigungsbereich (21) des Bioreaktorbehälters (1) in Eingriff ist.

11. Transportvorrichtung (31) zum Heben und Transportieren eines Bioreaktorbehälters (1) mit einer Handhabungseinrichtung (11), gemäß einem der Ansprüche 1 bis 9, wobei die Transportvorrichtung (31) aufweist:
- eine komplementäre Handhabungseinrichtung (33), welche ausgelegt ist mit der Handhabungseinrichtung (11) des Bioreaktorbehälters (1) in Eingriff zu gelangen;
- eine optionale Drehvorrichtung (35), durch welche die komplementäre Handhabungseinrichtung (33) um eine horizontale Achse (A) drehbar ist.

12. Verfahren zum Einsetzen eines zumindest bereichsweise flexiblen, zusammenstauchbaren Bioreaktorbehälters (1), gemäß einem der Ansprüche 1 bis 9, in eine Haltevorrichtung, wobei der Bioreaktorbehälter (1)
- eine Handhabungseinrichtung (11) und
- eine Sicherungseinrichtung (19), welche ein Entfalten des Bioreaktorbehälters (1) hemmt,
aufweist und wobei das Verfahren die Schritte umfaßt:
- Bereitstellen des Bioreaktorbehälters (1) in einem Transportzustand, in welchem der Behälter (1) zumindest bereichsweise entgegen einer Streckrichtung (S) zusammengestaucht ist;
- Befestigen der Handhabungseinrichtung (11) des Bioreaktorbehälters (1) an einer komplementären Handhabungseinrichtung (33) einer Transportvorrichtung (31), gemäß Anspruch 9;
- Verlagern der Transportvorrichtung (31), wobei der Bioreaktorbehälter (1) in die Haltevorrichtung zumindest bereichsweise eingebracht wird;
- Lösen der Sicherungseinrichtung (19), wodurch sich der Bioreaktorbehälter (1) zumindest bereichsweise entlang der Streckrichtung (S) entfaltet, um von dem Transportzustand in einen Anordnungszustand überführt zu werden;
- Lösen der Handhabungseinrichtung (11) von der komplementären Handhabungseinrichtung (33).

13. Verfahren gemäß Anspruch 12, wobei der Bioreaktorbehälter (1) ein entlang der Streckrichtung (S) längenvariables Element, ein Rührwerk (5), aufweist und wobei das Verfahren den weiteren Schritt umfaßt:
- Strecken des längenvariablen Elements entlang der Streckrichtung (S).

14. Verfahren gemäß Anspruch 12 oder 13, mit den Schritten:
- Stauchen und/oder Falten des Bioreaktorbehälters (1) nach dem Benutzen entgegen der Streckrichtung (S);
- Befestigen der Sicherungseinrichtung (19), wodurch der Bioreaktorbehälter (1) wieder in den Transportzustand überführt wird.

## Claims

1. A bioreactor container (1) for insertion into a holding apparatus, comprising:
- an at least partially flexible wall (3), which is shortened in a transport state opposite to a stretching direction (S) at least in part(s); and
- a handling device (11) connected to the wall (3), which is adapted to engage a complementary handling device (33) of a transport device (31); **characterized in that** the wall (3) is shortened in a transport state opposite to a stretching direction (S) at least in part(s); and
- that a securing device (19) is provided, which in a transport state inhibits stretching of the wall (3) of the bioreactor container (1) along the stretching direction (S), wherein the container (1) is extendable at least in part(s) along the stretching direction (S) when the securing device (19) is released.

2. The bioreactor container (1) according to claim 1, wherein the bioreactor container (1) has at least one securing device attachment region (21), with which an associated complementary securing device attachment region (23) of the securing device (19) is engageable to prevent displacement of the securing device (19) in a direction different to the stretching direction (S).

3. The bioreactor container (1) according to claim 2, wherein the at least one securing device attachment region (21) is arranged on a side of the wall (3) of the bioreactor container (1) opposite to the handling device (11) along the stretching direction (S), and
wherein the at least one securing device attachment region (21) is preferably formed on a bottom plate (25).

4. The bioreactor container (1) according to one of claims 1 to 3, wherein the securing device (19) is formed as a flexible, substantially non-stretchable band (19), and
wherein preferably the at least one complementary securing device attachment region (23) is formed as a hole (23) in the band (19).

5. The bioreactor container (1) according to claim 4, wherein the band (19) and the wall (3) of the bioreactor container (1) made of identical material.

6. The bioreactor container (1) according to one of claims 1 to 5, wherein the securing device (19) is attached on the wall (3) at least in part(s).

7. The bioreactor container (1) according to one of claims 1 to 3, wherein the securing device (19) is formed as at least one rigid connecting element, which surrounds the wall (3) of the bioreactor container (1) at least in part(s), and wherein the rigid connecting element is preferably non-detachably connected to the at least one securing device attachment region (21) and/or the bottom plate (25).

8. The bioreactor container (1) according to one of claims 1 to 7, wherein the at least one securing device (19) is formed to be reusable in order to secure the bioreactor container (1) after use and compression opposite to the stretching direction (S) in the transport state.

9. The bioreactor container according to one of claims 1 to 8, wherein the handling device (11) has a shaft coupling (17) to connect a shaft (9) disposed within the bioreactor container (1) with a drive arranged outside of the bioreactor container (1).

10. A use of a securing device (19) to hold a bioreactor container (1) having an at least partially flexible wall (3), which is shortened in a transport state opposite to a stretching direction (S) at least in part(s), in this transport state,
wherein the securing device (19) has at least one complementary securing device attachment region (23), which is engaged with an associated securing device attachment region (21) of the bioreactor container (1).

11. A transport device (31) for lifting and transporting a bioreactor container (1) with a handling device (11) according to one of claims 1 to 9, the transport device (31) comprising:
- a complementary handling device (33) formed to engage the handling device (11) of the bioreactor container (1);
- an optional turning device (35) by which the complementary handling device (33) is rotatable about a horizontal axis (A).

12. A method for inserting an at least partially flexible, compressible bioreactor container (1) according to one of claims 1 to 9 into a holding device, wherein the bioreactor container (1) includes
- a handling device (11) and
- a securing device (19), which inhibits unfolding of the bioreactor container (1),
and wherein the method comprises the steps of:
- providing the bioreactor container (1) in a transport state in which the container (1) is compressed at least in part(s) opposite to a stretching direction (S);
- attaching the handling device (11) of the bioreactor container (1) to a complementary handling device (33) of a transport device (31), according to claim 9;
- displacing the transport device (31), wherein the bioreactor container (1) is introduced into the holding device at least in part(s);
- loosening the securing device (19), whereby the bioreactor container (1) unfolds at least in part(s) along the stretching direction (S) in order to be transferred from the transport state into an arrangement state;
- releasing the handling device (11) from the complementary handling device (33).

13. The method according to claim 12, wherein the bioreactor container (1) includes a variable-length element along the stretching direction (S), an agitator (5), and wherein the method comprises the further step of:
- stretching the variable-length element along the stretching direction (S).

14. The method according to claim 12 or 13, comprising the steps of:
- compressing and/or folding the bioreactor container (1) after use opposite to the stretching direction (S);
- attaching the securing device (19), whereby the bioreactor container (1) is transferred back into the transport state.

## Revendications

1. Récipient de bioréacteur (1) pour l'insertion dans un dispositif de maintien comprenant :
- une paroi flexible au moins par région (3) qui est raccourcie au moins par région dans un état de transport à l'encontre d'un sens d'étirement (S) ; et
- un dispositif de manutention (11) connecté à la paroi (3) qui est conçu pour parvenir en engrènement avec un dispositif de manutention complémentaire (33) d'un dispositif de transport (31) ;
**caractérisé en ce que** la paroi (3) est raccourcie au moins par région dans un état de transport à l'encontre d'un sens d'étirement (S) ; et
- qu'un dispositif de sécurisation (19) est prévu, lequel empêche dans un état de transport un étirement de la paroi (3) du récipient de bioréacteur (1) le long du sens d'étirement (S), dans lequel le récipient (1) peut être rallongé au moins par région le long du sens d'étirement (S) lorsque le dispositif de sécurisation (19) est détaché.

2. Récipient de bioréacteur (1) selon la revendication 1, dans lequel le récipient de bioréacteur (1) présente au moins une région de fixation de dispositif de sécurisation (21) avec laquelle une région de fixation de dispositif de sécurisation complémentaire associée (23) du dispositif de sécurisation (19) peut être parvenue en engrènement pour empêcher un déplacement du dispositif de sécurisation (19) dans un sens différent du sens d'étirement (S).

3. Récipient de bioréacteur (1) selon la revendication 2, dans lequel l'au moins une région de fixation de dispositif de sécurisation (21) est disposée sur un côté de la paroi (3) du récipient de bioréacteur (1) qui fait face au dispositif de manutention (11) le long du sens d'étirement (S), et
dans lequel l'au moins une région de fixation de dispositif de sécurisation (21) est de préférence réalisée sur une plaque de fond (25).

4. Récipient de bioréacteur (1) selon une des revendications 1 à 3, dans lequel le dispositif de sécurisation (19) est réalisé en tant que ruban flexible (19) essentiellement non étirable, et
dans lequel l'au moins une région de fixation de dispositif de sécurisation complémentaire (23) est de préférence réalisée en tant que trou (23) dans le ruban (19).

5. Récipient de bioréacteur (1) selon la revendication 4, dans lequel le ruban (19) et la paroi (3) du récipient de bioréacteur (1) se composent d'un matériau identique.

6. Récipient de bioréacteur (1) selon une des revendications 1 à 5, dans lequel le dispositif de sécurisation (19) est fixé au moins par région à la paroi (3).

7. Récipient de bioréacteur (1) selon une des revendications 1 à 3, dans lequel le dispositif de sécurisation (19) est réalisé en tant qu'au moins un élément de connexion rigide qui entoure au moins par région la paroi (3) du récipient de bioréacteur (1), et
dans lequel l'élément de connexion rigide est de préférence connecté de manière permanente à l'au moins une région de fixation de dispositif de sécurisation (21) et/ou à la plaque de fond (25).

8. Récipient de bioréacteur (1) selon une des revendications 1 à 7, dans lequel l'au moins un dispositif de sécurisation (19) est réalisé de manière réutilisable pour
sécuriser le récipient de bioréacteur (1) dans l'état de transport après l'utilisation et le réaplatissement à l'encontre du sens d'étirement (S).

9. Récipient de bioréacteur selon une des revendications 1 à 8, dans lequel le dispositif de manutention (11) présente un accouplement d'arbre (17) pour connecter un arbre (9) disposé au sein du récipient de bioréacteur (1) à un entraînement disposé en dehors du récipient de bioréacteur (1).

10. Utilisation d'un dispositif de sécurisation (19) pour maintenir un récipient de bioréacteur (1) avec une paroi flexible au moins par région (3) qui est raccourcie au moins par région dans un état de transport à l'encontre d'un sens d'étirement (S) dans cet état de transport,
dans laquelle le dispositif de sécurisation (19) présente au moins une région de fixation de dispositif de sécurisation complémentaire (23) qui est en engrènement avec une région de fixation de dispositif de sécurisation associée (21) du récipient de bioréacteur (1).

11. Dispositif de transport (31) pour le levage et transport d'un récipient de bioréacteur (1) avec un dispositif de manutention (11), selon une des revendications 1 à 9, dans lequel le dispositif de transport (31) présente :
- un dispositif de manutention complémentaire (33) qui est conçu pour parvenir en engrènement avec le dispositif de manutention (11) du récipient de bioréacteur (1) ;
- un dispositif de rotation optionnel (35) par lequel le dispositif de manutention complémentaire (33) peut être tourné autour d'un axe horizontal (A).

12. Procédé d'insertion d'un récipient de bioréacteur (1) flexible au moins par région, pouvant être réaplati, selon une des revendications 1 à 9, dans un dispositif de maintien, dans lequel le récipient de bioréacteur (1) présente
- un dispositif de manutention (11) et
- un dispositif de sécurisation (19) qui empêche un dépliage du récipient de bioréacteur (1),
et dans lequel le procédé comprend les étapes de :
- mise à disposition du récipient de bioréacteur (1) dans un état de transport dans lequel le récipient (1) est réaplati au moins par région à l'encontre d'un sens d'étirement (S) ;
- fixation du dispositif de manutention (11) du récipient de bioréacteur (1) sur un dispositif de manutention complémentaire (33) d'un dispositif de transport (31), selon la revendication 9 ;
- déplacement du dispositif de transport (31), dans lequel le récipient de bioréacteur (1) est introduit au moins par région dans le dispositif de maintien ;
- détachement du dispositif de sécurisation (19), moyennant quoi le récipient de bioréacteur (1) se déplie au moins par région le long du sens d'étirement (S) pour être transféré de l'état de transport dans un état d'agencement ;
- détachement du dispositif de manutention (11) du dispositif de manutention complémentaire (33).

13. Procédé selon la revendication 12, dans lequel le récipient de bioréacteur (1) comprend un élément de longueur variable le long du sens d'étirement (S), un agitateur (5), et dans lequel le procédé comprend l'étape supplémentaire de :
- étirement de l'élément de longueur variable le long du sens d'étirement (S).

14. Procédé selon la revendication 12 ou 13, avec les étapes de :
- aplatissement et/ou pliage du récipient de bioréacteur (1) après l'utilisation à l'encontre du sens d'étirement (S) ;
- fixation du dispositif de sécurisation (19), moyennant quoi le récipient de bioréacteur (1) est de nouveau transféré dans l'état de transport.
